# EUROPEAN PATENT SPECIFICATION

(11) **EP 3 371 307 B1**
(45) Date of publication and mention of the grant of the patent: **04.12.2024**
(21) Application number: 16798334.5
(22) Date of filing: 07.11.2016
(51) Int. Cl.: C12N 9/24, C11D 3/386, C12P 21/06, A23J 3/34

(54) **PAENIBACILLUS AND BACILLUS SPP. MANNANASES**
PAENIBACILLUS UND BACILLUS SPP. MANNANASEN
MANNANASES DE PAENIBACILLUS ET BACILLUS SPP.

(30) Priority: 05.11.2015 US 201562251516 P; 13.01.2016 US 201662278383 P
(43) Date of publication of application: 12.09.2018
(73) Proprietor: Danisco US Inc., Palo Alto, California 94304 (US)
(72) Inventor: BAO, Kai, Palo Alto, California 94304 (US); FIORESI, Carol Marie, Palo Alto, California 94304 (US); FINAN, Dina, Palo Alto, California 94304 (US); ADAMS, Christian D., Palo Alto, California 94304 (US); ALEKSEYEV, Viktor Yuryevich, Palo Alto, California 94304 (US); BOTT, Richard R., Palo Alto, California 94304 (US); RIFE, Christopher L., Palo Alto, California 94304 (US)
(74) Representative: Mewburn Ellis LLP
(86) International application number: PCT/US2016/060850
(87) International publication number: WO 2017/079756

(56) References cited:
- WO-A1-01/64853
- WO-A1-2014/100018
- WO-A1-2015/022428
- WO-A2-2008/009673
- DATABASE Geneseq 10 March 2016 (2016-03-10), "Paenibacillus sp. A1 mature endo-beta-mannanase (PspMan4), SEQ 52."
- DATABASE Geneseq [online] 10 March 2016 (2016-03-10), "Paenibacillus sp. endo-beta-mannanase (PamMan3) precursor protein SEQ 62.", retrieved from EBI accession no. GSP:BCK93931 Database accession no. BCK93931
- DATABASE Geneseq [online] 21 January 2010 (2010-01-21), "Plant biomass degradation related protein SEQ ID NO:410.", retrieved from EBI accession no. GSP:AXR38272 Database accession no. AXR38272
- DATABASE REFSEQ [online] 11 January 2015 (2015-01-11), "endoglucanase (Paenibacillus polymyxa)", XP002766090, retrieved from EBI accession no. WP_039270469 Database accession no. WP_039270469
- DATABASE REFSEQ [online] 24 December 2014 (2014-12-24), "endoglucanase (Paenibacillus sp. FSL H7-689)", XP002766091, retrieved from EBI accession no. Refseq: WP_036608478 Database accession no. WP_036608478
- MUNI RAMMANNAGARI SUBHOSH CHANDRA: "Isolation, Purification and Characterization of a Thermostable [beta]-Mannanase from Paenibacillus sp. DZ3", HAN'GUG EUNG'YONG SAENGMYEONG HWA HAGHOEJI - JOURNAL OF THE KOREAN SOCIETY FOR APPLIED BIOLOGICAL CHEMISTRY, vol. 54, no. 3, 30 June 2011 (2011-06-30), Seoul, Korea, pages 325 - 331, XP055335373, ISSN: 1738-2203, DOI: 10.3839/jksabc.2011.052
- SAMRITI DHAWAN ET AL: "A [beta]-mannanase from Paenibacillus sp.: Optimization of production and its possible prebiotic potential", BIOTECHNOLOGY AND APPLIED BIOCHEMISTRY, vol. 63, no. 5, 12 October 2015 (2015-10-12), US, pages 669 - 678, XP055335366, ISSN: 0885-4513, DOI: 10.1002/bab.1419
- DATABASE Geneseq [online] 10 March 2016 (2016-03-10), "Paenibacillus sp. A1 mature endo-beta-mannanase (PspMan4), SEQ 52.", XP002766092, retrieved from EBI accession no. GSP:BCK93921 Database accession no. BCK93921

## Description

Disclosed herein are mannanases from *Paenibacillus or Bacillus spp,* polynucleotides encoding the mannanases, compositions containing the mannanases, and methods of use thereof. Compositions containing mannanases are suitable for use as detergents and for cleaning fabrics and hard surfaces, as well as in a variety of other industrial applications.

Mannanase enzymes, including endo-β-mannanases, have been employed in detergent cleaning compositions for the removal of gum stains by hydrolyzing mannans. A variety of mannans are found in nature, such as, for example, linear mannan, glucomannan, galactomannan, and glucogalactomannan. Each such mannan is comprised of polysaccharides that contain a β-1,4-linked backbone of mannose residues that may be substituted up to 33% with glucose residues (Yeoman et al., Adv Appl Microbiol, 70:1, 2010, Elsevier). In galactomannans or glucogalactomannnans, galactose residues are linked in alpha-1,6-linkages to the mannan backbone (Moreira and Filho, Appl Microbiol Biotechnol, 79:165, 2008). Therefore, hydrolysis of mannan to its component sugars requires endo-1,4-β-mannanases that hydrolyze the backbone linkages to generate short chain manno-oligosaccharides that are further degraded to monosaccharides by 1,4-β-mannosidases. Although mannanases, such as, for example, endo-β-mannanases have been known in the art of industrial enzymes, there remains a need for further mannanases that are suitable for particular conditions and uses.

WO 2015/022428 discloses methods for producing a coffee extract which comprises use of an enzyme having β-1,3-galactanase activity and to a coffee extract which comprises at least 20% based on the total weight of soluble coffee solids of total galactose.

Chandra et al. (J. Korean Soc. Appl. Biol. Chem. 54, 325-331, 2011) describes the isolation, purification and characterisation of a thermostable β-mannanase from *Paenibacillus* sp. DZ3 and describes its optimal temperature and pH for mannanase activity as well as its mannanase activity towards glucomannan and galactomannan.

Dhawan et al. (Biotechnol Appl Biochem. 63(5):669-678, 2016) describes the isolation of a thermotolerant bacterium *Paenibacillus thiaminolyticus* with an ability to produce extracellular β-mannanase and describes the optimization of culture conditions for the high-level production of β-mannanase.

WO 2016/007929 discloses certain endo-β-mannanases from *Paenibacillus and Bacillus spp.,* compositions comprises such endo-β-mannanases and methods of use thereof.

Variants, compositions and methods disclosed herein relate to recombinant glycosyl hydrolase family 5 (GH5) mannanases, or recombinant polypeptides or active fragments thereof. For example, the *Paenibacillus sp.* PspMan4 mannanase (SEQ ID NO:2), which is a wild-type mannanase more fully described in PCT/US15/40057 filed July 10, 2015 (subsequently published as WO2016/007929), is a GH5 mannanase that has the expected activity for a mannanase and displays high structural similarities with other GH5 members when the three dimensional structures are compared. The three-dimensional structures of the following two multiply substituted PspMan4 variants: PspMan118 (SEQ ID NO:6) and PspMan148 (SEQ ID NO:7) are homologous to the *Bacillus sp.* JAMB-602 (PDB entry 1WKY_A) (SEQ ID NO:10) and *B*. *agaradhaerens* (PDB entry 2WHL_A) (residues 30-330 of QSYEX6) (SEQ ID NO: 9) GH5 mannanase structures. The GH5 mannanases: BspMan5 (SEQ ID NO:16), WO2015022428-0015 (SEQ ID NO:8), residues 32-330 of US6566114-002 (SEQ ID NO:15) and residues 32-340 of US6566114-002 (SEQ ID NO:17) all have greater than 90% amino acid sequence identity to the amino acid sequence of 1WKY_A (SEQ ID NO: 10), and as a result many of the sites explored in the mannanase variants described herein have the same amino acid at each structurally corresponding position in, for example, the 2WHL_A (SEQ ID NO:9), 1WKY_A (SEQ ID NO: 10), PspMan4 (SEQ ID NO:2) and other NDL-clade mannanases, BspMan5 (SEQ ID NO:16),WO2015022428-0015 (SEQ ID NO:8), US6566114-002 (residues 32-330) (SEQ ID NO:15) and US6566114-002 (residues 32-340) (SEQ ID NO:17) amino acid sequences. Therefore, substitutions in these and other GH5 mannanases that are structurally similar to 2WHI, A (SEQ ID NO:9), 1WKY_A (SEQ ID NO:10), PspMan4 (SEQ ID NO:2) or other NDL-clade mannanases, BspMan5 (SEQ ID NO:16),WO2015022428-0015 (SEQ ID NO:8), US6566114-002(residues 32-330) (SEQ ID NO:15), and US6566114-002 (residues 32-340) (SEQ ID NO:17) mannanases are expected to confer the same improved performance and stability as those substitutions described herein in relation to reference polypeptides PspMan4 (SEQ ID NO:2), BspMan5 (SEQ ID NO:16), and US6566114-002 (residues 32-340) (SEQ ID NO:17).

Provided herein is a mannanase variant comprising an amino acid sequence comprising one or more variations versus SEQ ID NO: 2 selected from:
X19E, X38E, X67D, X129M, X168S, X184L, X225P, X244L, X258D, and X261R;
wherein X is any amino acid;
wherein the amino acid positions of said variant are numbered by correspondence with the amino acid sequence of SEQ ID NO:2;
wherein the mannanase variant has at least 90% identity to SEQ ID NO: 2, and wherein the mannanase variant has one or more improved property over a reference polypeptide having the amino acid sequence of SEQ ID NO: 2, wherein the improved property is selected from thermal stability, detergent stability, specific activity towards a mannan substrate, and cleaning performance as determined using the Microswatch Assay described in Example 1,
with the proviso that the mannanase variant does not have the amino acid sequence set forth as any one of SEQ ID NOs: 62, 63, 65, 66, 67, 80 and 82 in WO 2016/007929, as set forth in the claims.

The GH5_8 sub family of mannanases is more fully described in Aspeborg et al (2012), "Evolution, substrate specificity and subfamily classification of glycosyl hydrolase family 5 (GH5)", BMC Evolutionary Biology, 12:186. In yet an even still further embodiment, the mannanase variant has at least 91%, 92%, 93%, 94%, 95%, 96%, 97%, 98%, or 99% amino acid sequence identity with the amino acid sequence of SEQ ID NO: 2. In an even further embodiment, the mannanase variant or is a GH5 mannanase.

### DESCRIPTION OF THE DRAWINGS

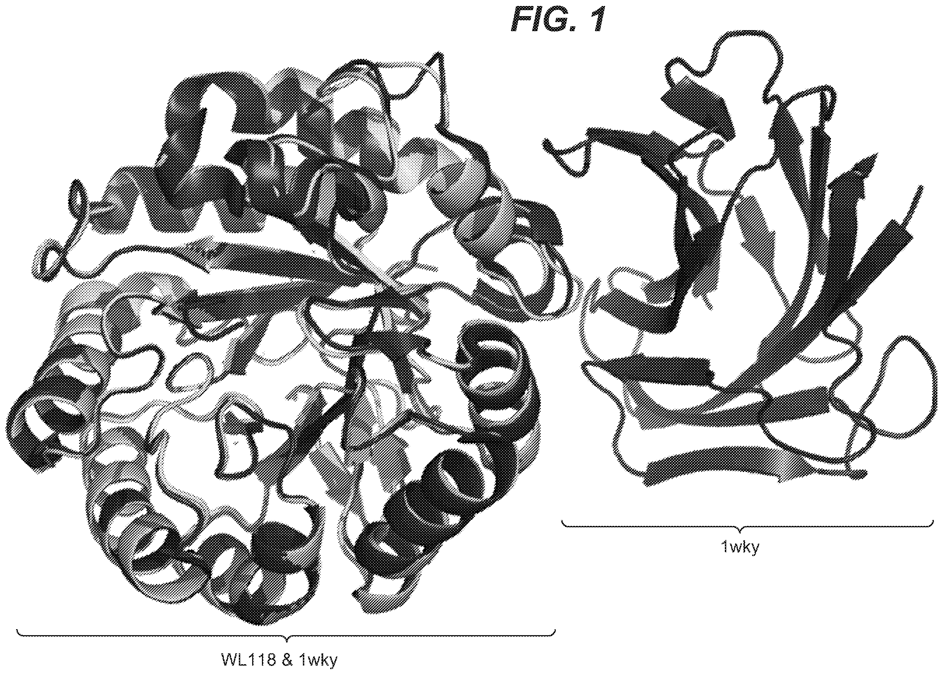

Figure 1 depicts a structural comparison of the 1WKY_A mannanase to the PspMan118 mannanase variant with the main chain of the 1WKY_A mannanase being shown in grey and the main chain of the PspMan118 mannanase being shown in black.

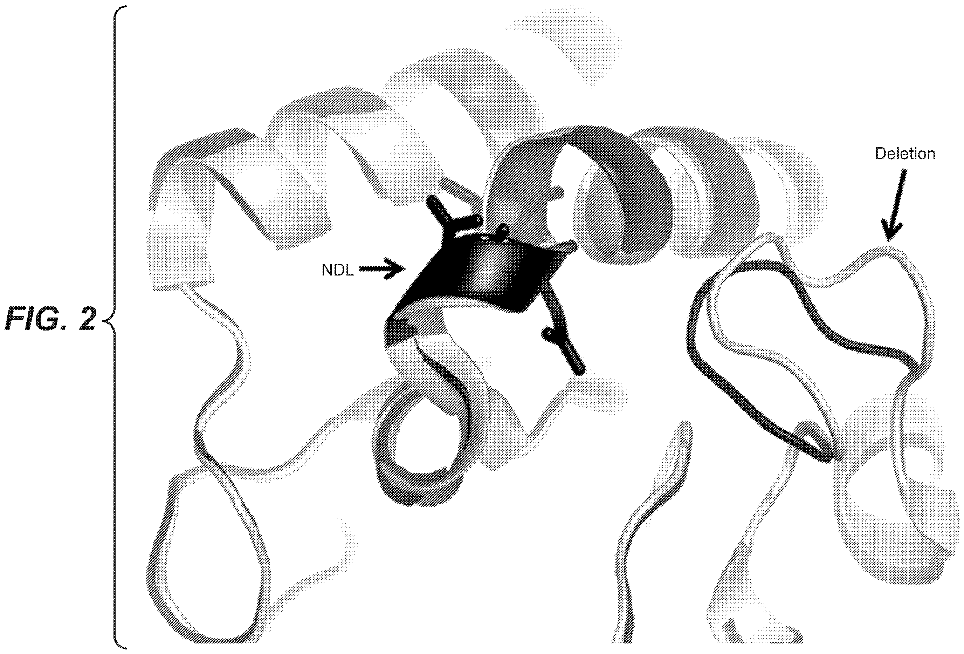

Figure 2 depicts a structural comparison of the PspMan118 and 1WKY_A structures in the region of the NDL and Deletion motifs of PspMan118.

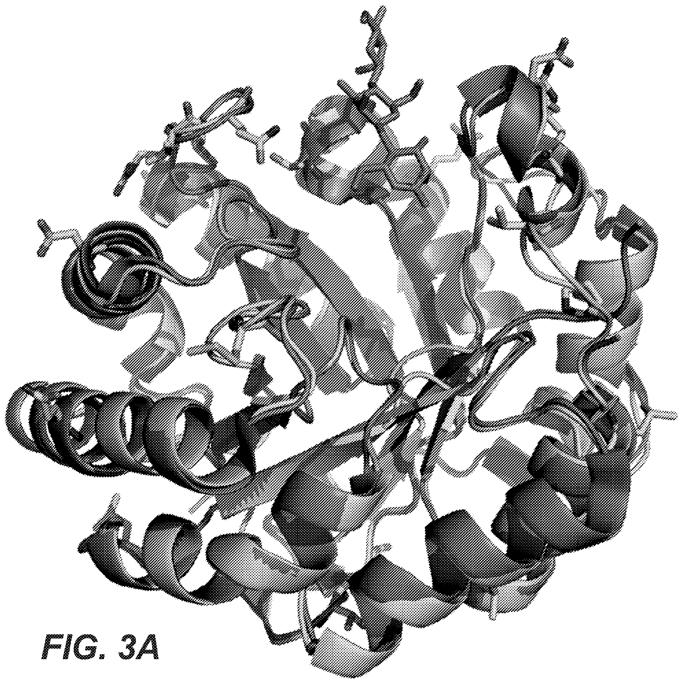

Figure 3A depicts a comparison of the main chain folding of the PspMan148 (black) and 2WHI, A (light gray) mannanases with the mannotriosyl moiety bound to 2WHL_A shown as gray sticks (to indicate the relative location of the substrate binding site) and the side chains of the eighteen amino acid substitutions present in PspMan148 shown as black stick figures.

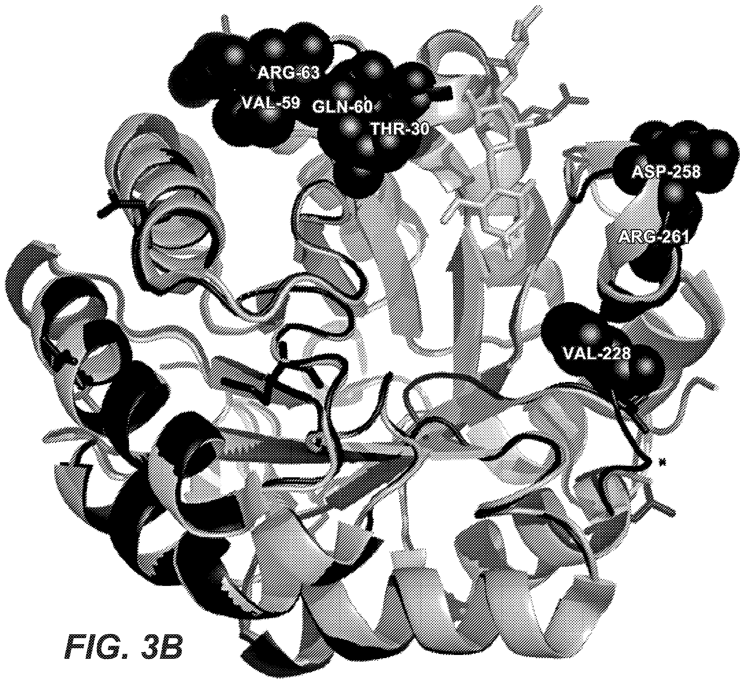

Figure 3B depicts a comparison of the main chain folding of the PspMan148 (black) and 2WHL_A (light gray) mannanases with the mannotriosyl moiety bound to 2WHL_A shown as gray sticks (to indicate the relative location of the substrate binding site) and the positions of the seven substitutions (S30T, S59V, L60Q, K63R, T228V, S258D and N261R) in PspMan148 around and near the substrate binding site shown as black spheres.

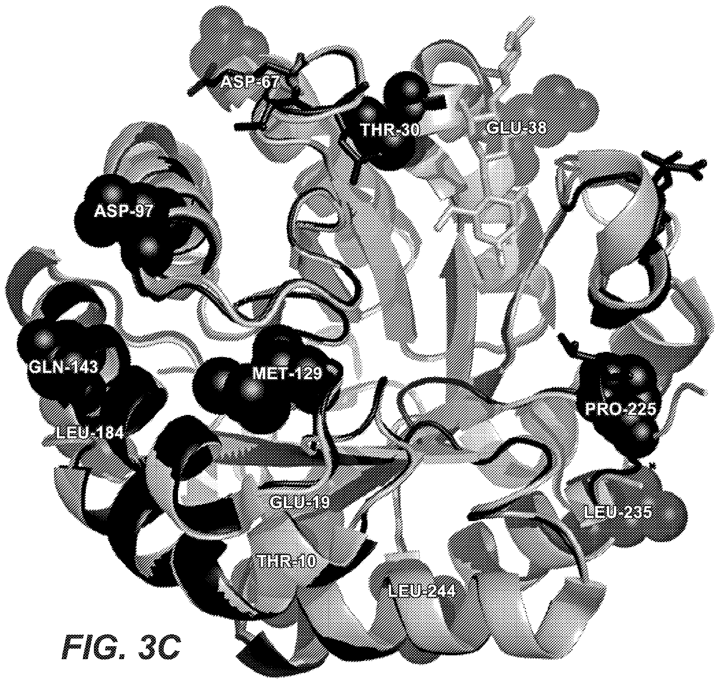

Figure 3C depicts a comparison of the main chain folding of the PspMan148 (black) and 2WHL_A (light gray) mannanases with the mannotriosyl moiety bound to 2WHL_A shown as gray sticks (to indicate the relative location of the substrate binding site) and the eleven surface substitutions in PspMan148 shown as black spheres.

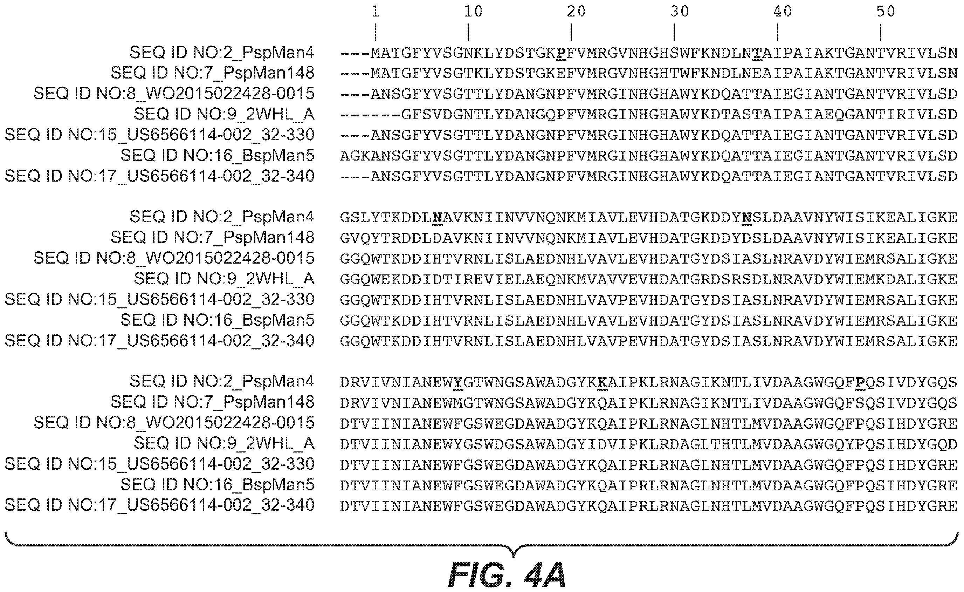

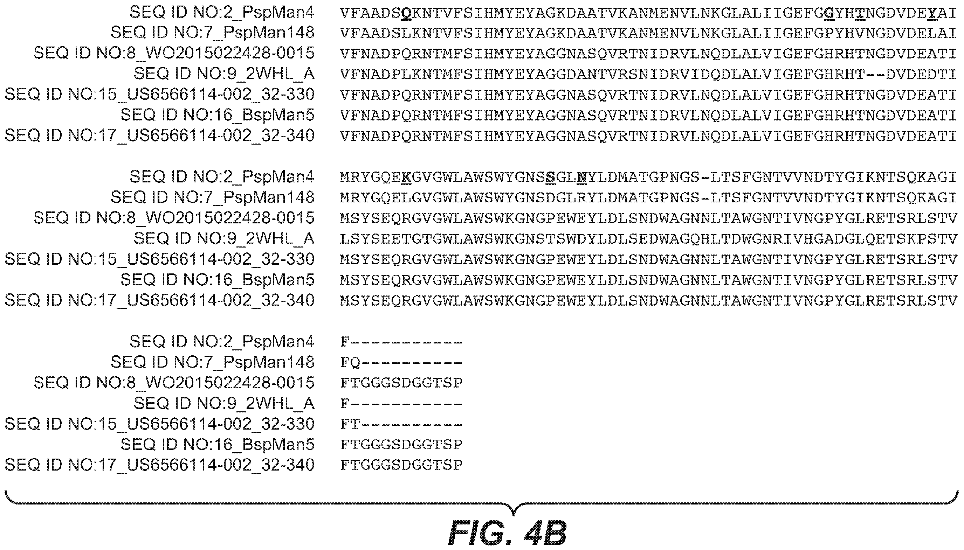

Figures 4A-B depict the multiple sequence alignment using MUSCLE software of the mannanase catalytic domains of PspMan4 (SEQ ID NO:2), PspMan148 (SEQ ID NO:7), BspMan5 (SEQ ID NO:16), US6566114-002 (residues 32-330)(SEQ ID NO:15), US6566114-002 (residues 32-340)(SEQ ID NO:17), WO2015022428-0015 (SEQ ID NO:8), and 2WHL_A (SEQ ID NO:9) with productive positions in PspMan4 being underlined and in bold font.

Described herein are endo-β-mannanases from *Paenibacillus* or *Bacillus spp.,* polynucleotides encoding such endo-β-mannanases, cleaning compositions containing such mannanases, and methods of use thereof. The *Paenibacillus* or *Bacillus spp.* endo-β-mannanases described herein may have glycosyl hydrolase activity and/or are stable in the presence of a cleaning composition and/or protease. These features of the endo-β-mannanases described herein make them well suited for use in a variety of cleaning and other industrial applications, for example, where the enzyme can hydrolyze mannans in the presence of surfactant, protease, and/or other components found in a detergent composition. The mannanase variants of the invention are set forth in the claims.

The following terms are defined for clarity. Terms and abbreviations not defined should be accorded their ordinary meaning as used in the art. For example, technical and scientific terms not defined herein have the same meaning as commonly understood by one of ordinary skill in the art to which this disclosure pertains *(See, e.g.,* Singleton and Sainsbury, Dictionary of Microbiology and Molecular Biology, 2d Ed., John Wiley and Sons, NY 1994; and Hale and Marham, The Harper Collins Dictionary of Biology, Harper Perennial, NY 1991).

The singular terms "a," "an," and "the" include the plural reference unless the context clearly indicates otherwise.

The terms "mannan endo-1,4-β-mannosidase," "endo-1,4-β-mannanase," "endo-β-1,4-mannase," "β-mannanase B," "β-1, 4-mannan 4-mannanohydrolase," "endo-β-mannanase," "β-D-mannanase," "1,4-β-D-mannan mannanohydrolase," or "endo-β-mannanase" (EC 3.2.1.78) refer to an enzyme capable of the random hydrolysis of 1,4-β-D-mannosidic linkages in mannans, galactomannans and glucomannans. Endo-1,4-β-mannanases are members of several families of glycosyl hydrolases, including GH26 and GH5. In particular, endo-β-mannanases constitute a group of polysaccharases that degrade mannans and denote enzymes that are capable of cleaving polyose chains containing mannose units *(i.e.,* are capable of cleaving glycosidic bonds in mannans, glucomannans, galactomannans and galactogluco-mannans). The "endo-β-mannanases" described herein may possess additional enzymatic activities (*e.g*., endo-1,4-β-glucanase, 1,4- β -mannosidase, and cellodextrinase activities).

The terms "mannanase," "mannosidic enzyme," "mannolytic enzyme," "mannanase enzyme," "mannanase polypeptides," or "mannanase proteins" refer to an enzyme, polypeptide, or protein that can degrade mannan. The mannanase enzyme may, for example, be an endo-β-mannanase, an exo-β-mannanase, or a glycosyl hydrolase. As used herein, mannanase activity may be determined according to any procedure known in the art (*See*, *e.g.,* Lever, Anal. Biochem, 47:273, 1972; Eriksson and Winell, Acta Chem. Scand., (1968), 22:1924; US 6602842; and WO9535362A1).

As used herein, "mannans" are polysaccharides having a backbone composed of β-1,4-linked mannose; "glucomannans" are polysaccharides having a backbone of more or less regularly alternating β-1,4 linked mannose and glucose; "galactomannans" and "galactoglucomannans" are mannans and glucomannans with alpha-1,6 linked galactose side-branches. These compounds may be acetylated. The degradation of galactomannans and galactoglucomannans is facilitated by full or partial removal of the galactose side-branches. Further, the degradation of the acetylated mannans, glucomannans, galactomannans and galactoglucomannans is facilitated by full or partial deacetylation. Acetyl groups can be removed by alkali or by mannan acetylesterases. The oligomers that are released from the mannanases or by a combination of mannanases and alpha-galactosidase and/or mannan acetyl esterases can be further degraded to release free maltose by β-mannosidase and/or β-glucosidase.

The term "modification" refers to any change or alteration in an amino acid sequence, including the substitution of an amino acid at the identified position of the amino acid sequence of interest with an amino acid that is different from the starting amino acid, deletion of an amino acid at the identified position of the amino acid sequence of interest, insertion of an amino acid at the identified position of the amino acid sequence of interest, replacement of an amino acid side chain in the amino acid sequence of interest, and or chemical modification of the amino acid sequence of interest.

The terms "catalytic activity" or "activity" describes quantitatively the conversion of a given substrate under defined reaction conditions. The term "residual activity" is defined as the ratio of the catalytic activity of the enzyme under a certain set of conditions to the catalytic activity under a different set of conditions. The term "specific activity" describes quantitatively the catalytic activity per amount of enzyme under defined reaction conditions.

The term "pH-stability" describes the ability of a protein to withstand a limited exposure to pH-values significantly deviating from the pH where its stability is optimal (*e.g*., more than one pH-unit above or below the pH-optimum), without losing its activity under conditions where its activity is measurable.

The term "detergent stability" refers to the stability of a specified detergent composition component (such as a hydrolytic enzyme) in a detergent composition mixture.

The term "perhydrolase" refers to an enzyme capable of catalyzing a reaction that results in the formation of a peracid suitable for applications such as cleaning, bleaching, and disinfecting.

The term "aqueous," as used in the phrases "aqueous composition" and "aqueous environment" refers to a composition that is made up of at least 50% water. An aqueous composition may contain at least 50%, 60%, 70%, 80%, 90%, 95%, 97%, 98%, or 99% water.

The term "surfactant" refers to any compound generally recognized in the art as having surface active qualities. Surfactants generally include anionic, cationic, nonionic, and zwitterionic compounds, which are further described, herein.

The term "surface property" is used in reference to electrostatic charge, as well as properties such as the hydrophobicity and hydrophilicity exhibited by the surface of a protein.

The term "chelator stability" refers to endo-β-mannanases of the present disclosure that retain a specified amount of enzymatic activity over a given period of time under conditions prevailing during the mannosidic, hydrolyzing, cleaning, or other process disclosed herein, for example while exposed to or contacted with chelating agents. In some embodiments, the mannanase retains at least about 50%, about 60%, about 70%, about 75%, about 80%, about 85%, about 90%, about 92%, about 95%, about 96%, about 97%, about 98%, or about 99% mannanase activity after contact with a chelating agent over a given time period, for example, at least about 10 minutes, about 20 minutes, about 40 minutes, about 60 minutes, about 100 minutes, etc.

The terms "thermal stability" and "thermostable" refer to mannanases that retain a specified amount of enzymatic activity after exposure to elevated temperatures over a given period of time under conditions prevailing during the mannosidic, hydrolyzing, cleaning, or other process, for example, while exposed to elevated temperatures. In some embodiments, the mannanase retains at least about 50%, about 60%, about 70%, about 75%, about 80%, about 85%, about 90%, about 92%, about 95%, about 96%, about 97%, about 98%, or about 99% mannanase activity after exposure to elevated temperatures, for example, at least about 50°C, about 55°C, about 60°C, about 65°C, or about 70°C, over a given time period, for example, at least about 5 minutes, 10 minutes, 15 minutes, 20 minutes, 30 minutes, 40 minutes, 50 minutes, 60 minutes, 120 minutes, 180 minutes, 240 minutes, 300 minutes, etc.

The term "cleaning activity" refers to the cleaning performance achieved by an endo-β-mannanase under conditions prevailing during the mannosidic, hydrolyzing, cleaning, or other process disclosed herein. In the mannanase variants claimed, cleaning performance is determined using the Microswatch Assay described in Example 1. Also described herein are various cleaning assays concerning enzyme sensitive stains arising from food products, household agents or personal care products. Some of these stains include, for example, ice cream, ketchup, BBQ sauce, mayonnaise, soups, chocolate milk, chocolate pudding, frozen desserts, shampoo, body lotion, sun protection products, toothpaste, locust bean gum, or guar gum as determined by various chromatographic, spectrophotometric or other quantitative methodologies after subjection of the stains to standard wash conditions. Exemplary assays include, but are not limited to those described in WO99/34011, US 6,605,458, and US 6,566,114, as well as those methods described in the Examples.

The terms "clean surface" and "clean textile" refer to a surface or textile respectively that has a percent stain removal of at least 10%, preferably at least 15%, 20%, 25%, 30%, 35%, or 40% of a soiled surface or textile.

The term "effective amount" when used in conjunction with a mannanase variant or recombinant polypeptide or active fragment thereof refers to the quantity of mannanase variant or recombinant polypeptide or active fragment thereof needed to achieve the desired level of enzymatic activity in the specified cleaning composition. Such effective amounts are readily ascertained by one of ordinary skill in the art and are based on many factors, such as the particular mannanase variant or recombinant polypeptide or active fragment thereof that is used, the cleaning application, the specific composition of the cleaning composition, and whether a liquid or dry (*e.g*., granular, bar, powder, solid, liquid, tablet, gel, paste, foam, sheet, or unit dose) composition is required.

The term "adjunct ingredient" when used in conjunction with a cleaning composition means any liquid, solid or gaseous material selected for the particular type of cleaning composition desired and the form of the product *(e.g.,* liquid, granule, powder, bar, paste, spray, tablet, gel, unit dose, sheet, or foam composition), which materials are also preferably compatible with the mannanase variant or recombinant polypeptide or active fragment thereof used in the composition. In some embodiments, granular compositions are in "compact" form, while in other embodiments, the liquid compositions are in a "concentrated" form.

The terms "cleaning compositions" and "cleaning formulations" refer to admixtures of chemical ingredients that find use in the removal of undesired compounds (*e.g*., soil or stains) from items or surfaces to be cleaned, such as, for example, fabric, dishes, contact lenses, solid surfaces, hair, skin, and teeth. The compositions or formulations may be in the form of a liquid, gel, granule, powder, bar, paste, spray tablet, gel, unit dose, sheet, or foam, depending on the surface or item to be cleaned and the desired form of the composition or formulation.

The terms "detergent composition" and "detergent formulation" refer to mixtures of chemical ingredients intended for use in a wash medium for the cleaning of soiled objects. Detergent compositions/formulations generally include at least one surfactant, and may optionally include hydrolytic enzymes, oxido-reductases, builders, bleaching agents, bleach activators, bluing agents, fluorescent dyes, caking inhibitors, masking agents, enzyme activators, antioxidants, and solubilizers.

The term "dishwashing composition" refers to all forms of compositions including, for example, granular, unit-dose, and liquid forms for cleaning dishware and cutlery. In some embodiments, the dishwashing composition is an "automatic dishwashing" composition that finds use in automatic dishwashing machines. The term "dishware" refers to dishes (*e.g*., plates, cups, glasses, bowls, and containers) and cutlery (*e.g*., utensils including, but not limited to spoons, knives, and forks) of any material, including but not limited to ceramics, plastics, metals, china, glass, and acrylics.

The term "bleaching" refers to the treatment of a material (*e.g*., fabric, laundry, pulp, etc.) or surface for a sufficient length of time and under appropriate pH and temperature conditions to effect a brightening (*i.e.,* whitening) and/or cleaning of the material. Examples of chemicals suitable for bleaching include but are not limited to ClO₂, H₂O₂, peracids, and NO₂.

The term "wash performance" of a mannanase variant or recombinant polypeptide or active fragment thereof refers to the contribution of the variant or recombinant polypeptide or active fragment thereof to washing that provides additional cleaning performance to the detergent composition. Wash performance is compared under relevant washing conditions. The term "relevant washing conditions" is used herein to indicate the conditions, particularly washing temperature, time, washing mechanics, suds concentration, type of detergent, and water hardness, actually used in households in a dish or laundry detergent market segment.

As used herein, the term "disinfecting" refers to the removal of contaminants from the surfaces, as well as the inhibition or killing of microbes on the surfaces of items.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, a preferred filler salt is sodium sulfate.

The term "fabric" refers to, for example, woven, knit, and non-woven material, as well as staple fibers and filaments that can be converted to, for example, yarns and woven, knit, and non-woven fabrics. The term encompasses material made from natural, as well as synthetic (*e.g*., manufactured) fibers.

A nucleic acid or polynucleotide is "isolated" when it is at least partially or completely separated from other components, including but not limited to, for example, other proteins, nucleic acids, and cells. Similarly, a polypeptide, protein or peptide is "isolated" when it is at least partially or completely separated from other components, including but not limited to, for example, other proteins, nucleic acids, and cells. On a molar basis, an isolated species is more abundant than are other species in a composition. For example, an isolated species may comprise at least about 60%, 65%, 70%, 75%, 80%, 85%, 90%, 91%, 92%, 93%, 94%, 95%, 96%, 97%, 98%, 99%, or 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, 65%, 70%, 75%, 80%, 85%, 90%, 91%, 92%, 93%, 94%, 95%, 96%, 97%, 98%, 99%, 99.5%, 99.6%, 99.7%, 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than in a starting composition.

As used herein, a "polypeptide" refers to a molecule comprising a plurality of amino acids linked through peptide bonds. The terms "polypeptide," "peptide," and "protein" are used interchangeably. Proteins may optionally be modified (*e.g*., glycosylated, phosphorylated, acylated, farnesylated, prenylated, and sulfonated) to add functionality. Where such amino acid sequences exhibit activity, they may be referred to as an "enzyme". The conventional one-letter or three-letter codes for amino acid residues are used, with amino acid sequences being presented in the standard amino-to-carboxy terminal orientation *(i.e.,* N→C).

The term "polynucleotide" encompasses DNA, RNA, heteroduplexes, and synthetic molecules capable of encoding a polypeptide. Nucleic acids may be single-stranded or doublestranded, and may have chemical modifications. The terms "nucleic acid" and "polynucleotide" are used interchangeably. Because the genetic code is degenerate, more than one codon may be used to encode a particular amino acid, and the present compositions and methods encompass nucleotide sequences which encode a particular amino acid sequence. Unless otherwise indicated, nucleic acid sequences are presented in a 5'-to-3' orientation.

The terms "wild-type" and "parental", with respect to a polypeptide, refer to a naturally-occurring polypeptide that does not include a man-made substitution, insertion, or deletion at one or more amino acid positions. Similarly, the terms "wild-type" and "parental", with respect to a polynucleotide, refer to a naturally-occurring polynucleotide that does not include a man-made substitution, insertion, or deletion at one or more nucleosides. However, note that a polynucleotide encoding a wild-type or parental polypeptide is not limited to a naturally-occurring polynucleotide, and encompasses any polynucleotide encoding the wild-type or parental polypeptide.

As used herein, the term "naturally-occurring" refers to anything (*e.g*., polypeptide or nucleic acid sequences) that is found in nature. Conversely, the term "non-naturally occurring" refers to anything that is not found in nature (*e.g*., recombinant nucleic acids and polypeptide sequences produced in the laboratory or modification of the wild-type sequence).

The term "reference", with respect to a polypeptide, refers to a naturally-occurring polypeptide that does not include a man-made substitution, insertion, or deletion at one or more amino acid positions, as well as a naturally-occurring or synthetic polypeptide that includes one or more man-made substitutions, insertions, or deletions at one or more amino acid positions. Similarly, the term "reference", with respect to a polynucleotide, refers to a naturally-occurring polynucleotide that does not include a man-made substitution, insertion, or deletion of one or more nucleosides, as well as a naturally-occurring or synthetic polynucleotide that includes one or more man-made substitutions, insertions, or deletions at one or more nucleosides. For example, a polynucleotide encoding a wild-type or parental polypeptide is not limited to a naturally-occurring polynucleotide, and encompasses any polynucleotide encoding the wild-type or parental polypeptide.

The term "variation(s)" when used in the phrase "one or more variations versus SEQ ID NO:2" encompasses each amino acid that is different from the amino acid present at the corresponding position in SEQ ID NO:2. For example, the sequence of the variant of interest is aligned with SEQ ID NO:2 according to the alignment set forth in Figures 4A-B and each position in the variant compared to SEQ ID NO:2 to identify the amino acids at each position that are different from the amino acid present at the corresponding positions in SEQ ID NO:2 and each amino acid that is different from the corresponding amino acid in SEQ ID NO:2 is a variation.

The one letter code "Z" identifies an insertion or deletion in a parent or reference amino acid sequence. For an insertion relative to the parent or reference sequence, the one letter code "Z" is on the left side of the position number and further includes a number (e.g., .01) before each amino acid being inserted therein to indicate the order of the insertions. For example, the insertion of a one amino acid, glutamine (Q), at position 298 would be depicted as "Z298.01Q"; the insertion of one amino acid, X (where X can be any amino acid) at position 298 would be depicted as "Z298.01X"; and the insertion of three amino acids alanine (A), serine (S) and tyrosine (Y) between position 87 and 88 would be depicted as "Z87.01A/Z87.02S/Z87.03Y". For a deletion, the one letter code "Z" is on the right side of the position number. For example, the deletion of an alanine (A) from position 100 would be depicted as A100Z. A combination of some the above insertions and deletions would be depicted as: "G87S/Z87.01A/Z87.02S/Z87.03Y/A100Z".

The amino acid substitutions described herein use one or more of following nomenclatures: position or starting amino acid:position:substituted amino acid(s). Reference to only a position encompasses any starting amino acid that may be present in a reference polypeptide, parent or benchmark molecule at that position and any amino acid with which such starting amino acid may be substituted (i.e., the substituted amino acid necessarily excludes the starting amino acid of such reference polypeptide, parent or benchmark molecule). Reference to a substituted amino acid may be further expressed as several substituted amino acids separated by a foreslash ("/"). For example, X130A/N-209-213 represents a three amino acid substitution combination, wherein X is any starting amino acid at position 130 that can be substituted with an alanine (A) or an asparagine (N); 209 represents a position where any starting amino acid can be substituted with an amino acid that is not the starting amino acid; and 213 represents a position where any starting amino acid can be substituted with an amino acid that is not the starting amino acid. By way of further example, S101F/G/H/T/V represents five possible substitutions at position 101, wherein the starting amino acid serine (S) can be substituted with a phenylalanine (F), glycine (G), histidine (H), threonine (T), or valine (V).

The term "mannanase variant" refers to a polypeptide that is derived from a reference polypeptide by the substitution, addition, or deletion, of one or more amino acids, typically by recombinant DNA techniques. A mannanase variant may differ from a reference polypeptide by a small number of amino acid residues and may be defined by the level of primary amino acid sequence homology/identity with the reference polypeptide over the length of the catalytic domain. For example, a mannanase variant has at least 59%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% amino acid sequence identity with a reference polypeptide. The reference polypeptide includes naturally occurring and recombinant mannanases within the GH5_8 sub family of mannanases (endo-1,4 β-mannosidases, EC 3.2.1.78). This GH5_8 sub family is more fully described in Aspeborg et al (2012), "Evolution, substrate specificity and subfamily classification of glycosyl hydrolase family 5 (GH5)", BMC Evolutionary Biology, 12:186. Exemplary GH5_8 bacterial mannanases include, for example, NDL-Clade mannanases, such as, for example, PspMan4 (SEQ ID NO:2); and other mannanases such as, for example, BspMan5 (SEQ ID NO:16) and variants thereof, *Bac. sp.* 1WKY_A (BAD99527.1)(SEQ ID NO:10), *B. agaradhaerens* 2WHI,_A (residues 30-330 of QSYEX6)(SEQ ID NO:9), WO2015022428-0015 (SEQ ID NO:8), residues 32-330 of US6566114-002 (SEQ ID NO:15), and residues 32-340 of US6566114-002 (SEQ ID NO:17). The NDL-Clade of mannanases is more fully described in International Patent Application No. PCT/US15/40057, filed July 10, 2015, which subsequently published as WO2016/007929.

The term "variant polynucleotide" refers to a polynucleotide that encodes a mannanase variant, has a specified degree of homology/identity with a parent polynucleotide, or hybridizes under stringent conditions to a parent polynucleotide or the complement thereof. For example, a variant polynucleotide has at least 59%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity with a parent polynucleotide.

Sequence identity may be determined using known programs such as BLAST, ALIGN, and CLUSTAL using standard parameters. *(See, e.g.,* Altschul etal. [1990] J. Mol. Biol. 215:403-410; Henikoff et al. [1989] Proc. Natl. Acad. Sci. USA 89:10915; Karin et al. [1993] Proc. Natl. Acad. Sci. USA 90:5873; and Higgins etal. [1988] Gene 73:237-244). Software for performing BLAST analyses is publicly available through the National Center for Biotechnology Information (NCBI). Databases may also be searched using FASTA (Pearson etal. [1988] Proc. Natl. Acad. Sci. USA 85:2444-2448). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another useful algorithm for comparison of multiple protein sequences is the MUSCLE program from Geneious software (Biomatters Ltd.) (Robert C. Edgar, "MUSCLE: multiple sequence alignment with high accuracy and high throughput", Nucl. Acids Res. (2004) 32 (5): 1792-1797).

The term "derived from" encompasses the terms "originated from," "obtained from," "obtainable from," "isolated from," and "created from" and generally indicates that one specified material find its origin in another specified material or has features that can be described with reference to the another specified material.

The term "hybridization" refers to the process by which a strand of nucleic acid joins with a complementary strand through base pairing, as known in the art.

The term "hybridization conditions" refers to the conditions under which hybridization reactions are conducted. These conditions are typically classified by degree of "stringency" of the conditions under which hybridization is measured. The degree of stringency can be based, for example, on the melting temperature (Tₘ) of the nucleic acid binding complex or probe. For example, "maximum stringency" typically occurs at about Tₘ-5°C (5°C below the Tₘ of the probe); "high stringency" at about 5-10°C below the Tₘ; "intermediate stringency" at about 10-20°C below the Tₘ of the probe; and "low stringency" at about 20-25°C below the Tₘ. Alternatively, or in addition, hybridization conditions can be based upon the salt or ionic strength conditions of hybridization and/or one or more stringency washes, *e.g*., 6X SSC = very low stringency; 3X SSC = low to medium stringency; 1X SSC = medium stringency; and 0.5X SSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe. For applications requiring high selectivity, it is typically desirable to use relatively stringent conditions to form the hybrids (e.g., relatively low salt and/or high temperature conditions are used).

The terms "substantially similar" and "substantially identical" in the context of at least two nucleic acids or polypeptides means that a polynucleotide or polypeptide comprises either a sequence that has at least about 90%, 91%, 92%, 93%, 94%, 95%, 96%, 97%, 98%, or 99% identity to a parent or reference sequence, or a sequence that includes amino acid substitutions, insertions, deletions, or modifications made only to circumvent the present description without adding functionality.

The term "expression vector" refers to a DNA construct containing a DNA sequence that encodes the specified polypeptide and is operably linked to a suitable control sequence capable of effecting the expression of the polypeptides in a suitable host. Such control sequences include a promoter to effect transcription, an optional operator sequence to control such transcription, a sequence encoding suitable mRNA ribosome binding sites, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself.

The term "recombinant" refers to genetic material (*i.e.,* nucleic acids, the polypeptides they encode, and vectors and cells comprising such polynucleotides) that has been modified to alter its sequence or expression characteristics, such as by mutating the coding sequence to produce an altered polypeptide, fusing the coding sequence to that of another gene, placing a gene under the control of a different promoter, expressing a gene in a heterologous organism, expressing a gene at a decreased or elevated levels, expressing a gene conditionally or constitutively in manner different from its natural expression profile, and the like. Generally, recombinant nucleic acids, polypeptides, and cells based thereon, have been manipulated by man such that they are not identical to related nucleic acids, polypeptides, and cells found in nature.

The term "signal sequence" refers to a sequence of amino acids bound to the N-terminal portion of a polypeptide, and which facilitates the secretion of the mature form of the protein from the cell. The mature form of the extracellular protein lacks the signal sequence which is cleaved off during the secretion process.

The terms "selective marker" or "selectable marker" refer to a gene capable of expression in a host cell that allows for ease of selection of those hosts containing an introduced nucleic acid or vector. Examples of selectable markers include but are not limited to antimicrobial substances (e.g., hygromycin, bleomycin, or chloramphenicol) and/or genes that confer a metabolic advantage, such as a nutritional advantage, on the host cell. The term "selectable gene product" refers to a gene that encodes an enzymatic activity that confers resistance to an antibiotic or drug upon the cell in which the selectable marker is expressed.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

The term "host cells" generally refers to prokaryotic or eukaryotic hosts which are transformed or transfected with vectors constructed using recombinant DNA techniques known in the art. Transformed host cells are capable of either replicating vectors encoding the protein variants or expressing the desired protein variant. In the case of vectors which encode the pre- or pro-form of the protein variant, such variants, when expressed, are typically secreted from the host cell into the host cell medium.

The term "introduced" in the context of inserting a nucleic acid sequence into a cell, means transformation, transduction, or transfection. Means of transformation include protoplast transformation, calcium chloride precipitation, electroporation, naked DNA, and the like as known in the art. *(See,* Chang and Cohen [1979] Mol. Gen. Genet. 168: 111-115; Smith et al. [1986] Appl. Env. Microbiol. 51:634; and the review article by Ferrari et al., in Harwood, Bacillus, Plenum Publishing Corporation, pp. 57-72, 1989).

The term "about" when used in connection with a numerical value refers to a range of - 10% to +10% of the numerical value. For instance, the phrase a "pH value of about 6" refers to pH values of from 5.4 to 6.6.

Any headings used herein are provided for convenience and should not be construed as limitations. The description included under one heading may apply to the specification as a whole.

Variants, compositions and methods disclosed herein relate to a recombinant mannanase, or a recombinant polypeptide or an active fragment thereof comprising one or more substitutions at one or more positions, wherein such variants are generated through conventional molecular biology techniques (see, e.g., Sambrook et al, Molecular Cloning: Cold Spring Harbor Laboratory Press).

The mannanase variant of the invention is set forth in the claims. An embodiment is directed to a mannanase variant comprising an amino acid sequence comprising one or more substitutions at one or more positions selected from P19E, T38E, N67D, Y129M, Q184L, K244L, S258D, and N261R; wherein the amino acid positions of the variant or recombinant polypeptide or active fragment thereof are numbered by correspondence with the amino acid sequence of SEQ ID NO:2, as set forth in the claims.

Another embodiment is directed to an NDL-Clade of mannanases comprising one or more mannanase variants set forth in the claims wherein said variant further comprises one or more motifs selected from a: WXₐKNDLXXAI (SEQ ID NO:11) motif at positions 31-40, wherein Xₐ is F or Y and X is any amino acid ("Motif 1"); LDXXXGPXGXLT (SEQ ID NO:12) motif at positions 263-274, wherein X is any amino acid ("Deletion Motif 1"); LDX₁V/AT/AGPX₂GX₃LT (SEQ ID NO:13) motif at positions 263-274, wherein X₁ is an M or L, X₂ is N, A or S and X₃ is S, T or N ("Deletion Motif 2"); and LDM/LATGPN/AGS/TLT (SEQ ID NO:14) motif at positions 263-274 ("Deletion Motif 3"), wherein the amino acid positions of the variant or recombinant polypeptide or active fragment thereof are numbered by correspondence with the amino acid sequence of SEQ ID NO:2. A yet further embodiment is directed to an NDL-Clade of mannanases comprising one or more mannanase variants set forth in the claims, wherein said variant further comprises one or more motifs selected from a: WXₐKNDLXXAI (SEQ ID NO:11) motif at positions 31-40, wherein Xₐ is F or Y and X is any amino acid; LDXXXGPXGXLT (SEQ ID NO:12) motif at positions 263-274, wherein X is any amino acid; LDX₁V/AT/AGPX₂GX₃LT (SEQ ID NO:13) motif at positions 263-274, wherein X₁ is an M or L, X₂ is N, A or S and X₃ is S, T or N; and LDM/LATGPN/AG S/TLT (SEQ ID NO:14) motif at positions 263-274, wherein the amino acid positions of the variant or recombinant polypeptide or active fragment thereof are numbered by correspondence with the amino acid sequence of SEQ ID NO:2, with the proviso that the variant is not ACU308431, ETT37549, WP_036608478, WP_036670707, WP_017688745, WP_053782127, PamMan2, PamMan3, PtuMan2, AAX87003, WP_046227931, WP_024633848, PpaMan2, WP_017813111, PspMan9, AEX60762, WP_046214462, YP_003868989, YP_003944884, WP_017427981, AAX87002, WP_009593769, YP_006190599, or WP_019912481.

A further embodiment is directed to an NDL-Clade of mannanases comprising one or more mannanase variants set forth in the claims, wherein said variant further comprises a WXₐKNDLXXAI (SEQ ID NO:11) motif at positions 31-40, wherein Xₐ is F and X is any amino acid, wherein the amino acid positions of the variant are numbered by correspondence with the amino acid sequence of SEQ ID NO:2, with the proviso that the variant, or recombinant polypeptide or active fragment thereof is not ACU308431, ETT37549, WP_036608478, WP_036670707, WP_017688745, WP_053782127, WP_024633848, AAX87003, or AEX60762. A still further embodiment is directed to an NDL-Clade of mannanases comprising one or more mannanase variants set forth in the claims, wherein said variant further comprises a WXₐKNDLXXAI (SEQ ID NO:11) motif at positions 31-40, wherein Xₐ is F and X is any amino acid, wherein the amino acid positions of the variant are numbered by correspondence with the amino acid sequence of SEQ ID NO:2, with the proviso that the variant is not ACU308431, ETT37549, WP_036608478, WP_036670707, WP_017688745, WP_053782127, WP_024633848, AAX87003, AEX60762, PamMan2, PamMan3, PtuMan2, PpaMan2, or PspMan9.

In a still further embodiment, the NDL-Clade of mannanases comprises one or more mannanase variants set forth in the claims, wherein said variant further comprises a LDX₁V/AT/AGPX₂GX₃LT (SEQ ID NO:13) or LDM/LATGPN/AGS/TLT (SEQ ID NO:14) motif at positions 263-274, wherein X₁ is an M; X₂ is N, A or S; and X₃ is S, T or N, wherein the amino acid positions of the variant are numbered by correspondence with the amino acid sequence of SEQ ID NO:2, with the proviso that the variant thereof is not ACU30843, ETT37549, WP_036608478, WP_036670707, WP_017688745, or WP_046214462. In yet a still further embodiment, the NDL-Clade of mannanases comprises one or more mannanase variants set forth in the claims, wherein said variant further comprises a LDX₁V/AT/AGPX₂GX₃LT (SEQ ID NO:13) or LDM/LATGPN/AGS/TLT (SEQ ID NO:14) motif at positions 263-274, wherein X₁ is an M; X₂ is N, A or S; and X₃ is S, T or N, wherein the amino acid positions of the variant are numbered by correspondence with the amino acid sequence of SEQ ID NO:2, with the proviso that the variant is not ACU30843, ETT37549, WP_036608478, WP_036670707, WP_017688745, WP_046214462, or PamMan2. In a still further embodiment, the NDL-Clade of mannanases comprises one or more mannanase variants set forth in the claims, wherein said variant further comprises (i) a WXₐKNDLXXAI (SEQ ID NO: 11) motif at positions 31-40, wherein Xₐ is F and X is any amino acid, and (ii) a LDX₁V/AT/AGPX₂GX₃LT (SEQ ID NO: 13) or LDM/LATGPN/AGS/TLT (SEQ ID NO: 14) motif at positions 263-274, wherein X₁ is an M; X₂ is N, A or S; and X₃ is S, T or N, wherein the amino acid positions of the variant are numbered by correspondence with the amino acid sequence of SEQ ID NO:2, with the proviso that the variant is not ACU30843, ETT37549, WP_036608478, WP_036670707, or WP_017688745. In an even still further embodiment, the NDL-Clade of mannanases comprises one or more mannanase variants set forth in the claims, wherein said variant further comprises (i) a WXₐKNDLXXAI (SEQ ID NO: 11) motif at positions 31-40, wherein Xₐ is F and X is any amino acid, and (ii) a LDX₁V/AT/AGPX₂GX₃LT (SEQ ID NO:13) or LDM/LATGPN/AGS/TLT (SEQ ID NO:14) motif at positions 263-274, wherein X₁ is an M; X₂ is N, A or S; and X₃ is S, T or N, wherein the amino acid positions of the variant are numbered by correspondence with the amino acid sequence of SEQ ID NO:2, with the proviso that the variant, or recombinant polypeptide or active fragment thereof is not ACU30843, ETT37549, WP_036608478, WP_036670707, WP_017688745, or PamMan2.

Another embodiment is directed to a mannanase variant comprising an amino acid sequence having at least 91%, 92%, 93%, 94%, 95%, 96%, 97%, 98%, or 99% amino acid sequence identity to the amino acid sequence of SEQ ID NO:2, as set forth in the claims.

In some embodiments, the mannanase are isolated. In other embodiments, the mannanase variants described herein are endo-β-mannanases. In further embodiments, the mannanase variants described herein have mannanase activity. In still other embodiments, the mannanase variants described herein have mannanase activity in the presence of a surfactant. In some embodiments, the mannanase activity is activity on mannan gum, locust bean gum galactomannan, and/or konjac glucomannan. In additional embodiments, the mannanase variants described herein have cleaning activity in a detergent composition. Still other embodiments are directed to mannanase variants that have mannanase activity in the presence of a protease. Further embodiments are directed to mannanase variants that hydrolyze a substrate selected from the group consisting of guar gum, locust bean gum, and combinations thereof. In some embodiments, the mannanase variants described herein do not comprise a carbohydrate-binding module.

In some embodiments, the mannanase variant has enzymatic activity over a broad range of pH conditions. In certain embodiments, the mannanase variant has enzymatic activity from a pH of about 4.0 to about 11.0. In further embodiments, the mannanase variants thereof have at least 50%, 60%, 70%, 80%, 90%, 95%, or 100% mannanase activity at a pH of from about 4.0 to about 11.0, about 4.5 to about 9.0, about 5.5 to about 8.5, or about 6.0 to about 7.5.

In a still further embodiment, the mannanase variants have mannanase activity at a temperature ranging from 20°C to about 90°C, about 30°C to about 80°C, about 20°C to about 50°C, or about 30°C to about 66°C. In certain embodiments, the mannanase variants have at least 50%, 60%, 70%, 80%, 90%, 95%, or 100% mannanase activity at a temperature range from about 20°C to about 90°C, about 30°C to about 80°C, about 20°C to about 50°C, or about 30°C to about 66°C.

Yet still further embodiments are directed to mannanase variants described herein, wherein the variant or recombinant polypeptide or active fragment thereof retains at least 70% of its maximal mannanase activity at a pH range of 4.5-9.0, 5.5-8.5, or 6.0-7.5. Some embodiments are directed to mannanase variants described herein, wherein the variant retains at least 70% of its maximal mannanase activity at a pH above 3.0, 3.5, 4.0 or 4.5 or at a pH below 9.0, 9.5, or 10.0. In some embodiments, the mannanase variant retains at least 10%, 20%, 30%, 40% or 50% residual mannanase activity at a temperature of from about 20-70°C, about 30-70°C, about 40-70°C, about 45-65°C, about 50-60°C, about 60-70°C, or about 60°C. In even further embodiments, the mannanase variant retains at least 70% of its maximal mannanase activity at a temperature range of about 40-70°C, about 45-75°C, about 45-65°C, about 50-60°C, or about 60-70°C. In other embodiments, the mannanase variant retains at least 70% of its maximal mannanase activity at a temperature above 20°C, 25°C, 30°C, 35°C, or 40°C or at a temperature below 60°C, 65°C, 70°C, 75°C, or 80°C. In still further embodiments, the amount of maximal mannanase activity retained is determined over a time period of 5 minutes.

In certain other embodiments, the mannanase variants described herein include substitutions that do not substantially affect the structure and/or function of the polypeptide. Exemplary substitutions are conservative mutations, as summarized in Table I.

**Table I. Amino Acid Substitutions**

| **Original Residue** | **Code** | **Acceptable Substitutions** |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, beta-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

In some embodiments, the mannanase variants have 1,4-β-D-mannosidic hydrolase activity, which includes mannanase, endo-1,4-β-D-mannanase, exo-1,4-β-D-mannanase galactomannanase, and/or glucomannanase activity. 1,4-β-D-mannosidic hydrolase activity can be determined and measured using the assays described herein, or by other assays known in the art. In some embodiments, a polypeptide of the present invention has activity in the presence of a detergent composition.

In some embodiments, the mannanase variants described herein are produced as an N-and/or C-terminal fusion protein, for example, to aid in extraction, detection and/or purification and/or to add functional properties to the variant. Examples of fusion protein partners include, but are not limited to, glutathione-S-transferase (GST), 6XHis, GAL4 (DNA binding and/or transcriptional activation domains), FLAG, MYC, BCE103 (WO 2010/044786), or other tags well known to anyone skilled in the art. In some embodiments, a proteolytic cleavage site is provided between the fusion protein partner and the protein sequence of interest to allow removal of fusion protein sequences. Preferably, the fusion protein does not hinder the activity of the mannanase variants described herein.

In some embodiments, the mannanase variants described herein are fused to a functional domain including a leader peptide, propeptide, one or more binding domain (modules) and/or a catalytic domain. Suitable binding domains include, but are not limited to, carbohydrate-binding modules (CBM) of various specificities, providing increased affinity to carbohydrate components present during the application of the mannanase variants or recombinant polypeptides or active fragments thereof described herein. As described herein, the CBM and catalytic domain of a polypeptide of the present invention are operably linked.

A CBM is defined as a contiguous amino acid sequence within a carbohydrate-active enzyme with a discreet fold having carbohydrate-binding activity. A few exceptions are CBMs in cellulosomal scaffold in proteins and rare instances of independent putative CBMs. The requirement of CBMs existing as modules within larger enzymes sets this class of carbohydrate-binding proteins apart from other non-catalytic sugar binding proteins such as lectins and sugar transport proteins. CBMs were previously classified as cellulose-binding domains (CBDs) based on the initial discovery of several modules that bound cellulose (Tomme et al., Eur J Biochem, 170:575-581, 1988; and Gilkes et al., J Biol Chem, 263:10401-10407, 1988). However, additional modules in carbohydrate-active enzymes are continually being found that bind carbohydrates other than cellulose, yet otherwise meet the CBM criteria, hence the need to reclassify these polypeptides using more inclusive terminology. Previous classification of cellulose-binding domains was based on amino acid similarity. Groupings of CBDs were called "Types" and numbered with roman numerals (e.g. Type I or Type II CBDs). In keeping with the glycoside hydrolase classification, these groupings are now called families and numbered with Arabic numerals. Families 1 to 13 are the same as Types I to XIII (Tomme et al., in Enzymatic Degradation of Insoluble Polysaccharides (Saddler, J.N. & Penner, M., eds.), Cellulose-binding domains: classification and properties, pp. 142-163, American Chemical Society, Washington, 1995). A detailed review on the structure and binding modes of CBMs can be found in Boraston et al., Biochem J, 382:769-81, 2004. The family classification of CBMs is expected to aid in the identification of CBMs, predict binding specificity, aid in identifying functional residues, reveal evolutionary relationships, and possibly be predictive of polypeptide folds. Because the fold of proteins is better conserved than their sequences, some of the CBM families can be grouped into superfamilies or clans. The current CBM families are 1-63. CBDs are found at the N-and C-termini of proteins or are internal. Enzyme hybrids are known in the art (See *e.g*., WO9000609 and WO9516782) and may be prepared by transforming into a host cell a DNA construct comprising at least a fragment of DNA encoding the cellulose-binding domain ligated, with or without a linker, to a DNA sequence encoding a mannanase variant or recombinant polypeptide or active fragment thereof described herein and growing the host cell to express the fused gene.

Enzyme hybrids may be described by the following formula:
CBM-MR-X or X-MR-CBM, wherein CBM is the N-terminal or the C-terminal region of an amino acid sequence corresponding to at least the carbohydrate-binding module; MR is the middle region (the linker), and may be a bond, or a short linking group of from about 2 to about 100 carbon atoms, from about 2 to about 40 carbon atoms, from about 2 to about 100 amino acids, or from about 2 to about 40 amino acids; and X is an N-terminal or C-terminal region of a mannanase variant or recombinant polypeptide or active fragment thereof described herein that has mannanase catalytic activity. In addition, a mannanase may contain more than one CBM or other module(s)/domain(s) of non-glycolytic function. The terms "module" and "domain" are used interchangeably in the present disclosure.

Further non-limiting examples of catalytic domains include: cellulases; hemicellulases, such as xylanase; exo-mannanases; glucanases; arabinases; galactosidases; pectinases; and/or other activities such as proteases, lipases, acid phosphatases and/or others or functional fragments thereof. Fusion proteins are optionally linked to a mannanase variant or recombinant polypeptide or active fragment thereof described herein through a linker sequence that simply joins the mannanase variant or recombinant polypeptide or active fragment thereof and the fusion domain without significantly affecting the properties of either component, or the linker optionally has a functional importance for the intended application.

Alternatively, the mannanase variants or recombinant polypeptides or active fragments thereof described herein are used in conjunction with one or more additional proteins of interest. Non-limiting examples of proteins of interest include: acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases and/or other enzymes.

In other embodiments, a mannanase variant is fused to a signal peptide for directing the extracellular secretion of the variant or polypeptide or active fragment thereof. For example, in certain embodiments, the signal peptide is the native signal peptide of the mannanase described herein. In other embodiments, the signal peptide is a non-native signal peptide such as the *B*. *subtilis* AprE signal peptide.

In some embodiments, a polypeptide of the present invention is expressed in a heterologous organism, *i.e.,* an organism other than *Paenibacillus spp.* Exemplary heterologous organisms are Gram(+) bacteria such as *B. subtilis, B. licheniformis, B. lentus, B. brevis, Geobacillus* (formerly *Bacillus*) *stearothermophilus, B. alkalophilus, B. amyloliquefaciens, B. coagulans, B. circulans, B. lautus, B. megaterium, B. thuringiensis, S. lividans,* or *S. murinus;* Gram(-) bacteria such as *E. coli;* yeast such as *Saccharomyces spp.* or *Schizosaccharomyces spp., e.g. S. cerevisiae;* and filamentous fungi such as *Aspergillus spp., e.g., A. oryzae* or *A. niger,* and *T. reesei.* Methods for transforming nucleic acids into these organisms are well known in the art. A suitable procedure for transformation of *Aspergillus* host cells is described in EP238023.

In particular embodiments, a mannanase variant described herein is expressed in a heterologous organism as a secreted polypeptide, in which case, the compositions and method encompass a method for expressing the variant as a secreted polypeptide in a heterologous organism.

Further embodiments are directed to methods of producing a mannanase variant described herein comprising: stably transforming a host cell with an expression vector comprising a polynucleotide encoding the mannanase variant; culturing the transformed host cell under suitable conditions to produce the mannanase variant; and recovering the mannanase variant.

Yet another embodiment is directed to a polynucleotide that encodes a mannanase variant described herein. In one aspect, the polynucleotide is contained in an expression vector contained in a heterologous organism, such as those identified, herein. The polynucleotide may be operably-linked to regulatory elements (*e.g*., a promoter, terminator, enhancer, and the like) to assist in expressing the encoded variants or recombinant polypeptides or active fragments thereof described herein.

Some embodiments are directed to a polynucleotide that encodes a variant set forth in the claims, which has at least 90%, 91%, 92%, 93%, 94%, 95%, 96%, 97%, 98%, or 99% amino acid sequence identity to the amino acid sequence of SEQ ID NO:2. In some embodiments, the polynucleotide is codon-optimized for expression in a different host, mutated to introduce cloning sites, or otherwise altered to add functionality.

In some embodiments, the polynucleotide that encodes a mannanase variant described herein is fused downstream of a coding sequence of a signal peptide that directs the extracellular secretion of variant. Expression vectors may be provided in a heterologous host cell suitable for expressing a variant or recombinant polypeptide or active fragment thereof described herein, or suitable for propagating the expression vector prior to introducing it into a suitable host cell.

In some embodiments, a polynucleotide that encodes a variant hybridizes to the polynucleotide of SEQ ID NO:1 or the complement thereof under specified hybridization conditions. Exemplary conditions are stringent condition and highly stringent conditions, which are described, herein.

DNA that encodes a mannanase variant or recombinant polypeptide or active fragment thereof described herein can be chemically synthesized from published sequences or obtained directly from host cells harboring the gene *(e.g.,* by cDNA library screening or PCR amplification). In some embodiments, a polynucleotide is included in an expression cassette and/or cloned into a suitable expression vector by standard molecular cloning techniques. Such expression cassettes or vectors contain sequences that assist initiation and termination of transcription (*e.g*., promoters and terminators), and generally contain a selectable marker.

The expression cassette or vector is introduced into a suitable expression host cell, which then expresses the corresponding mannanase variant or recombinant polypeptide or active fragment thereof described herein. Particularly suitable expression hosts are bacterial expression host genera including *Escherichia (e.g., E. coli), Pseudomonas (e.g., P. fluorescens or P. stutzerei), Proteus (e.g., P. mirabilis), Ralstonia (e.g., R. eutropha), Streptomyces, Staphylococcus (e.g., S. carnosus), Lactococcus (e.g., L. lactis), or Bacillus (subtilis, megaterium, licheniformis, etc.*)*.* Also particularly suitable are yeast expression hosts such as *S. cerevisiae, S. pombe, Y. lipolytica, H. polymorpha, K. lactis or P. pastoris.* Especially suited are fungal expression hosts such as *C.lucknowense, Aspergillus (e.g., A. oryzae, A. niger, A. nidulans, etc.)* or *T. reesei.* Also suited are mammalian expression hosts such as mouse (*e.g.,* NS0), Chinese Hamster Ovary (CHO) or Baby Hamster Kidney (BHK) cell lines. Other eukaryotic hosts such as insect cells or viral expression systems (*e.g*., bacteriophages such as M13, T7 phage or Lambda, or viruses such as Baculovirus) are also suitable for producing a mannanase variant or recombinant polypeptide or active fragment thereof described herein.

Promoters and/or signal sequences associated with secreted proteins in a particular host of interest are candidates for use in the heterologous production and secretion of mannanases in that host or in other hosts. As an example, in filamentous fungal systems, the promoters that drive the genes for cellobiohydrolase I (cbh1), glucoamylase A (glaA), TAKA-amylase (amyA), xylanase (exlA), the gpd-promoter cbh1, cbhll, endoglucanase genes EGI-EGV, Cel61B, Cel74A, egl1-egl5, gpd promoter, Pgk1, pki1, EF-1alpha, tef1, cDNA1 and hex1 are particularly suitable and can be derived from a number of different organisms (*e.g., A. niger, T. reesei, A. oryzae, A. awamori* and *A. nidulans*)*.* In some embodiments, the polynucleotide is recombinantly associated with a polynucleotide encoding a suitable homologous or heterologous signal sequence that leads to secretion of a mannanase variant described herein into the extracellular (or periplasmic) space, thereby allowing direct detection of enzyme activity in the cell supernatant (or periplasmic space or lysate). Particularly suitable signal sequences for E. *coli,* other Gram negative bacteria and other organisms known in the art include those that drive expression of the HlyA, DsbA, Pbp, PhoA, PelB, OmpA, OmpT or M13 phage Gill genes. For *B. subtilis,* Gram-positive organisms and other organisms known in the art, particularly suitable signal sequences further include those that drive expression of AprE, NprB, Mpr, AmyA, AmyE, Blac, SacB, and for *S. cerevisiae* or other yeast, include the killer toxin, Bar1, Suc2, Mating factor alpha, Inu1A or Ggplp signal sequence. Signal sequences can be cleaved by a number of signal peptidases, thus removing them from the rest of the expressed protein. In some embodiments, the rest of the polypeptide is expressed alone or as a fusion with other peptides, tags or proteins located at the N- or C-terminus (e.g., 6XHis, HA or FLAG tags). Suitable fusions include tags, peptides or proteins that facilitate affinity purification or detection (e.g., BCE103, 6XHis, HA, chitin binding protein, thioredoxin or FLAG tags), as well as those that facilitate expression, secretion or processing of the target mannanase. Suitable processing sites include enterokinase, STE13, Kex2 or other protease cleavage sites for cleavage *in vivo* or *in vitro.*

A mannanase variant or recombinant polypeptide or active fragment thereof described herein can be introduced into expression host cells by a number of transformation methods including, but not limited to, electroporation, lipid-assisted transformation or transfection ("lipofection"), chemically mediated transfection (e.g., CaCl and/or CaP), lithium acetate-mediated transformation (e.g., of host-cell protoplasts), biolistic "gene gun" transformation, PEG-mediated transformation (e.g., of host-cell protoplasts), protoplast fusion *(e.g.,* using bacterial or eukaryotic protoplasts), liposome-mediated transformation, *Agrobacterium tumefaciens,* adenovirus or other viral or phage transformation or transduction.

Alternatively, a mannanase variant or recombinant polypeptide or active fragment thereof described herein can be expressed intracellularly. Optionally, after intracellular expression of the enzyme variants, or secretion into the periplasmic space using signal sequences such as those mentioned above, a permeabilisation or lysis step can be used to release the polypeptide into the supernatant. The disruption of the membrane barrier is effected by the use of mechanical means such as ultrasonic waves, pressure treatment (French press), cavitation or the use of membrane-digesting enzymes such as lysozyme or enzyme mixtures. As a further alternative, the polynucleotides encoding a mannanase variant or recombinant polypeptide or active fragment thereof described herein can be expressed by use of a suitable cell-free expression system. In cell-free systems, the polynucleotide of interest is typically transcribed with the assistance of a promoter, but ligation to form a circular expression vector is optional. In other embodiments, RNA is exogenously added or generated without transcription and translated in cell free systems.

Another embodiment is directed to a cleaning composition comprising a mannanase variant and methods for using such compositions in cleaning applications. Cleaning applications include, but are not limited to, laundry or textile cleaning, laundry or textile softening, dishwashing (manual and automatic), stain pre-treatment, and the like. Particular applications are those where mannans (*e.g.*, locust bean gum, guar gum, etc.) are a component of the soils or stains to be removed.

Cleaning compositions typically include an effective amount of a mannanase variant or recombinant polypeptide or active fragment thereof described herein, *e.g.,* at least 0.0001 weight percent, from about 0.0001 to about 1, from about 0.001 to about 0.5, from about 0.01 to about 0.1 weight percent, or even from about 0.1 to about 1 weight percent, or more. An effective amount of a mannanase variant or recombinant polypeptide or active fragment thereof in the cleaning composition results in the mannanase variant or recombinant polypeptide or active fragment thereof having enzymatic activity sufficient to hydrolyze a mannan-containing substrate, such as locust bean gum, guar gum, or combinations thereof.

Some embodiments are directed to a cleaning composition in a form selected from powder, liquid, granular, bar, solid, semi-solid, gel, paste, emulsion, tablet, capsule, unit dose, sheet, and foam. In some embodiments, the cleaning composition is a detergent composition. In other embodiments, the cleaning composition or detergent composition is selected from a laundry detergent, a fabric softening detergent, a dishwashing detergent, and a hard-surface cleaning detergent.

Unless otherwise noted, all component or composition levels provided herein are made in reference to the active level of that component or composition, and are exclusive of impurities, for example, residual solvents or by-products, which may be present in commercially available sources. Enzyme component weights are based on total active protein. All percentages and ratios are calculated by weight unless otherwise indicated. All percentages and ratios are calculated based on the total composition unless otherwise indicated. In exemplified detergent compositions, the enzymes levels are expressed by pure enzyme by weight of the total composition and unless otherwise specified, the detergent ingredients are expressed by weight of the total compositions.

In some embodiments, the cleaning compositions described herein further comprise a surfactant. In some embodiments, the surfactant is selected from a non-ionic, ampholytic, semi-polar, anionic, cationic, zwitterionic, and combinations and mixtures thereof. In yet a further embodiment, the surfactant is selected from an anionic surfactant, a cationic surfactant, a zwitterionic surfactant, and combinations thereof. In some embodiments, the cleaning compositions described herein comprise from about 0.1% to about 60%, about 1% to about 50%, or about 5% to about 40% surfactant by weight of the composition. Exemplary surfactants include, but are not limited to sodium dodecylbenzene sulfonate, C12-14 pareth-7, C12-15 pareth-7, sodium C12-15 pareth sulfate, C14-15 pareth-4, sodium laureth sulfate (e.g., Steol CS-370), sodium hydrogenated cocoate, C₁₂ ethoxylates (Alfonic 1012-6, Hetoxol LA7, Hetoxol LA4), sodium alkyl benzene sulfonates (e.g., Nacconol 90G), and combinations and mixtures thereof. Anionic surfactants include but are not limited to linear alkylbenzenesulfonate (LAS), alpha-olefinsulfonate (AOS), alkyl sulfate (fatty alcohol sulfate) (AS), alcohol ethoxysulfate (AEOS or AES), secondary alkanesulfonates (SAS), alpha-sulfo fatty acid methyl esters, alkyl- or alkenylsuccinic acid, or soap. Nonionic surfactants include but are not limited to alcohol ethoxylate (AEO or AE), carboxylated alcohol ethoxylates, nonylphenol ethoxylate, alkylpolyglycoside, alkyldimethylamine oxide, ethoxylated fatty acid monoethanolamide, fatty acid monoethanolamide, polyhydroxy alkyl fatty acid amide *(e.g.,* as described in WO9206154), polyoxyethylene esters of fatty acids, polyoxyethylene sorbitan esters (e.g., TWEENs), polyoxyethylene alcohols, polyoxyethylene isoalcohols, polyoxyethylene ethers (e.g., TRITONs and BRIJ), polyoxyethylene esters, polyoxyethylene-p-tert-octylphenols or octylphenyl-ethylene oxide condensates *(e.g.,* NONIDET P40), ethylene oxide condensates with fatty alcohols (*e.g.,* LUBROL), polyoxyethylene nonylphenols, polyalkylene glycols (SYNPERONIC F108), sugar-based surfactants (e.g., glycopyranosides, thioglycopyranosides), and combinations and mixtures thereof.

In a further embodiment, the detergent compositions disclosed herein further comprise a surfactant mixture that includes, but is not limited to 5-15% anionic surfactants, < 5% nonionic surfactants, cationic surfactants, phosphonates, soap, enzymes, perfume, butylphenyl methylpropionate, geraniol, zeolite, polycarboxylates, hexyl cinnamal, limonene, cationic surfactants, citronellol, and benzisothiazolinone.

The cleaning compositions described herein may additionally include one or more detergent builders or builder systems, a complexing agent, a polymer, a bleaching system, a stabilizer, a foam booster, a suds suppressor, an anti-corrosion agent, a soil-suspending agent, an anti-soil redeposition agent, a dye, a bactericide, a hydrotope, a tarnish inhibitor, an optical brightener, a fabric conditioner, and a perfume. The cleaning compositions described herein may also include additional enzymes selected from proteases, amylases, cellulases, lipases, pectin degrading enzymes, xyloglucanases, or additional carboxylic ester hydrolases.

In some embodiments, the cleaning composition described herein further comprises from about 1%, from about 3% to about 60% or even from about 5% to about 40% builder by weight of the cleaning composition. Builders may include, but are not limited to, the alkali metals, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1,3,5-trihydroxy benzene-2,4,6-trisulphonic acid, and carboxymethyloxysuccinic acid, the various alkali metals, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof.

In some embodiments, the builders form water-soluble hardness ion complexes (e.g., sequestering builders), such as citrates and polyphosphates (e.g., sodium tripolyphosphate and sodium tripolyphospate hexahydrate, potassium tripolyphosphate, and mixed sodium and potassium tripolyphosphate, etc.). Any suitable builder can find use in the compositions described herein, including those known in the art *(See, e.g.,* EP2100949).

As indicated herein, in some embodiments, the cleaning compositions described herein further comprise an adjunct ingredient including, but not limited to surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, hydrolyzable surfactants, solvents, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents *(See, e.g.,* US 6610642, US6605458, US5705464, US5710115, US5698504, US5695679, US5686014, and US 5646101). In some embodiments, one or more adjunct is incorporated for example, to assist or enhance cleaning performance, for treatment of the substrate to be cleaned, or to modify the aesthetics of the cleaning composition as is the case with perfumes, colorants, dyes or the like. Any such adjunct ingredient is in addition to the mannanase variant or recombinant polypeptide or active fragment thereof described herein. The precise nature of these additional components, and levels of incorporation thereof, will depend on the physical form of the composition and the nature of the cleaning operation for which it is to be used.

In embodiments in which one or more adjunct ingredient is not compatible with the mannanase variant or recombinant polypeptide or active fragment thereof, suitable methods can be employed to keep the cleaning adjunct ingredient and mannanases separated *(i.e.,* not in contact with each other) until combination of the two components is appropriate. Such separation methods include any suitable method known in the art (e.g., gelcaps, encapsulation, tablets, physical separation, etc.). The specific selection of suitable adjunct ingredients is readily made by considering the surface, item, or fabric to be cleaned, and the desired form of the composition for the cleaning conditions during use (e.g., through the wash detergent use).

The cleaning compositions described herein are advantageously employed for example, in laundry applications, hard surface cleaning, dishwashing applications, as well as cosmetic applications. Furthermore, the polypeptides of the present invention may find use in granular and liquid compositions.

A mannanase variant or recombinant polypeptide or active fragment thereof described herein may also find use in cleaning additive products. In some embodiments, the additive is packaged in a dosage form suitable for addition to a cleaning process. In some embodiments, the additive is packaged in a dosage form for addition to a cleaning process where a source of peroxygen is employed and increased bleaching effectiveness is desired. Any suitable single unit dosage form finds use with the present disclosure, including but not limited to pills, tablets, gelcaps, or other single unit dosage form such as pre-measured powders or liquids. In some embodiments, filler(s) or carrier material(s) are included to increase the volume of such compositions. Suitable filler or carrier materials include, but are not limited to various salts of sulfate, carbonate, and silicate as well as talc, clay, and the like. Suitable filler or carrier materials for liquid compositions include, but are not limited to water or low molecular weight primary and secondary alcohols including polyols and diols. Examples of such alcohols include, but are not limited to methanol, ethanol, propanol, and isopropanol. In some embodiments, the compositions contain from about 5% to about 90% of such materials. Acidic fillers find use to reduce pH. Alternatively, in some embodiments, the cleaning additive includes one or more adjunct ingredients.

In one embodiment, the cleaning composition or cleaning additive contains an effective amount of a mannanase variant described herein, optionally in combination with other mannanases and/or additional enzymes. In certain embodiments, the additional enzymes include, but are not limited to, at least one enzyme selected from acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, metalloproteases, oxidases, pectate lyases, pectin acetyl esterases, pectinases, pentosanases, perhydrolases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, and combinations thereof. In further embodiments, the cleaning compositions or cleaning additives described herein further comprise a protease and/or amylase.

The cleaning compositions herein are typically formulated such that, during use in aqueous cleaning operations, the wash water will have a pH of from about 3.0 to about 11. Liquid product formulations are typically formulated to have a neat pH from about 5.0 to about 9.0. Granular laundry products are typically formulated to have a pH from about 8.0 to about 11.0. Techniques for controlling pH at recommended usage levels include the use of buffers, alkalis, acids, etc., and are well known to those skilled in the art.

Suitable low pH cleaning compositions typically have a neat pH of from about 3.0 to about 5.0 or even from about 3.5 to about 4.5. Low pH cleaning compositions are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine, or hydrochloric acid, to provide such cleaning composition with a neat pH of from about 3.0 to about 5.0. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of the composition wherein the solvent is distilled water. In these embodiments, all pH measurements are taken at 20°C, unless otherwise indicated.

Suitable high pH cleaning compositions typically have a neat pH of from about 9.0 to about 11.0, or even a neat pH of from 9.5 to 10.5. Such cleaning compositions typically comprise a sufficient amount of a pH modifier, such as sodium hydroxide, monoethanolamine, or hydrochloric acid, to provide such cleaning composition with a neat pH of from about 9.0 to about 11.0. Such compositions typically comprise at least one base-stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, the mannanase variant is in the form of an encapsulated particle to protect it from other components of the granular composition during storage. In addition, encapsulation is also a means of controlling the availability of the mannanase variant or recombinant polypeptide or active fragment thereof during the cleaning process. In some embodiments, encapsulation enhances the performance of the mannanase variant and/or additional enzymes. In this regard, the mannanase variant is encapsulated with any suitable encapsulating material known in the art. Typically, the encapsulating material is water-soluble and/or water-dispersible. In some embodiments, the encapsulating material has a glass transition temperature (Tg) of 0°C or higher. Glass transition temperature is described in more detail in WO9711151. The encapsulating material is typically selected from carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch *(See, e.g.,* EP0922499 and US4977252, US5354559, and US5935826). In some embodiments, the encapsulating material is a microsphere made from plastic such as thermoplastics, acrylonitrile, methacrylonitrile, polyacrylonitrile, polymethacrylonitrile, and mixtures thereof; commercially available microspheres include, but are not limited to those supplied by EXPANCEL^{®} (Stockviksverken, Sweden), and PM6545, PM6550, PM7220, PM7228, EXTENDOSPHERES^{®}, LUXSIL^{®}, Q-CEL^{®}, and SPHERICEL^{®} (PQ Corp., Valley Forge, PA).

The term "granular composition" refers to a conglomeration of discrete solid, macroscopic particles. Powders are a special class of granular material due to their small particle size, which makes them more cohesive and more easily suspended.

Concentrations of detergent compositions in typical wash solutions throughout the world vary from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

In some embodiments, the detergent compositions described herein may be utilized at a temperature of from about 10°C to about 60°C, or from about 20°C to about 60°C, or from about 30°C to about 60°C, from about 40°C to about 60°C, from about 40°C to about 55°C, or all ranges within 10°C to 60°C. In some embodiments, the detergent compositions described herein are used in "cold water washing" at temperatures of from about 10°C to about 40°C, or from about 20°C to about 30°C, from about 15°C to about 25°C, from about 15°C to about 35°C, or all ranges within 10°C to 40°C.

As a further example, different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

**Table II. Water Hardness Levels**

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca²⁺/Mg²⁺ (e.g., about 15 grains per gallon mixed Ca²⁺/Mg²⁺). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca²⁺/Mg²⁺.

In some embodiments, a mannanase variant described herein is comparable in wash performance to commercially available mannanases. In some embodiments, a mannanase variant described herein exhibits enhanced wash performance as compared to commercially available mannanases. In some embodiments, a mannanase variant exhibits enhanced oxidative stability, enhanced thermal stability, enhanced cleaning capabilities under various conditions, and/or enhanced chelator stability. In addition, a mannanase variant described herein may find use in cleaning compositions that do not include detergents, again either alone or in combination with builders and stabilizers.

In addition to the mannanase variants or recombinant polypeptides or active fragments thereof described herein, any other suitable mannanase may find use in the compositions described herein either alone or in combination with the variants or recombinant polypeptides or active fragments thereof described herein. Suitable mannanases include, but are not limited to, mannanases of the GH26 family of glycosyl hydrolases, mannanases of the GH5 family of glycosyl hydrolases, acidic mannanases, neutral mannanases, and alkaline mannanases. Examples of alkaline mannanases include those described in US6060299, US6566114, and US 6602842; and WO9535362, WO9964573, WO9964619, and WO2015022428. Additionally, suitable mannanases include, but are not limited to those of animal, plant, fungal, or bacterial origin. Chemically or genetically modified mutants are encompassed by the present disclosure.

Examples of useful mannanases include *Bacillus* endo-β-mannanases such as *B. subtilis* endo-β-mannanase *(See, e.g.,* US 6,060,299 and WO9964573), *Bacillus sp.* I633 endo-β-mannanase *(See, e.g.,* US 6,566,114 and WO9964619), *Bacillus* sp. AAI12 endo-β-mannanase *(See, e.g.,* US 6,566,114 and WO9964619), *B. sp.* AA349 endo-β-mannanase *(See, e.g.,* US 6,566,114 and WO9964619), *B. agaradhaerens* NCIMB 40482 endo-β-mannanase *(See, e.g.,* US 6,566,114 and WO9964619), *B. halodurans* endo-β-mannanase, *B. clausii* endo-β-mannanase *(See, e.g.,* US 6,566,114 and WO9964619), *B. licheniformis* endo-β-mannanase *(See, e.g.,* US 6,566,114 and WO9964619A1), *Humicola* endo-β-mannanases such as *H. insolens* endo-β-mannanase *(See, e.g.,* US 6,566,114 and WO9964619), and *Caldocellulosiruptor* endo-β-mannanases such as C. sp. endo-β-mannanase *(See, e.g.,* US 6,566,114 and WO9964619).

Furthermore, a number of identified mannanases (*i.e.,* endo-β-mannanases and exo-β-mannanases) find use in some embodiments of the present disclosure, including but not limited to *A. bisporus* mannanase *(See,* Tang et al., [2001] Appl. Environ. Microbiol. 67:2298-2303), *A. tamarii* mannanase *(See,* Civas et al., [1984] Biochem. J. 219:857-863), *A. aculeatus* mannanase *(See,* Christgau et al., [1994] Biochem. Mol. Biol. Int. 33:917-925), *A. awamori* mannanase *(See,* Setati et al., [2001] Protein Express Purif. 21:105-114), *A. fumigatus* mannanase *(See,* Puchart et al., [2004] Biochimica et biophysica Acta. 1674:239-250), *A. niger* mannanase *(See,* Ademark et al., [1998] J. Biotechnol. 63:199-210), *A. oryzae* NRRL mannanase *(See,* Regalado et al., [2000] J. Sci. Food Agric. 80:1343-1350), *A. sulphureus* mannanase *(See,* Chen et al., [2007] J. Biotechnol. 128(3):452-461), *A. terrus* mannanase *(See,* Huang et al., [2007] Wei Sheng Wu Xue Bao. 47(2): 280-284), *Paenibacillus and Bacillus spp.* mannanase *(See,* US 6,376,445.), *Bacillus* AM001 mannanase *(See,* Akino et al., [1989] Arch. Microbiol. 152:10-15), *B. brevis* mannanase *(See,* Araujo and Ward, [1990] J. Appl. Bacteriol. 68:253-261), *B. circulans* K-1 mannanase *(See,* Yoshida etal., [1998] Biosci. Biotechnol. Biochem. 62(3):514-520), *B. polymyxa* mannanase *(See,* Araujo and Ward, [1990] J. Appl. Bacteriol. 68:253-261), *Bacillus sp* JAMB-750 mannanase *(See,* Hatada et al., [2005] Extremophiles. 9:497-500), *Bacillus sp.* M50 mannanase *(See,* Chen et al., [2000] Wei Sheng Wu Xue Bao. 40:62-68), *Bacillus sp.* N 16-5 mannanase *(See,* Yanhe et al., [2004] Extremophiles 8:447-454), *B. stearothermophilus* mannanase *(See,* Talbot and Sygusch, [1990] Appl. Environ. Microbiol. 56: 3505-3510), *B. subtilis* mannanase *(See,* Mendoza et al., [1994] World J. Microbiol. Biotechnol. 10:51-54), *B. subtilis* B36 mannanase (Li etal., [2006] Z. Naturforsch (C). 61:840-846), *B. subtilis* BM9602 mannanase *(See,* Cui et al., [1999] Wei Sheng Wu Xue Bao. 39(1):60-63), *B. subtilis SA-22* mannanase *(See,* Sun etal., [2003] Sheng Wu Gong Cheng Xue Bao. 19(3):327-330), *B. subtilis168* mannanase *(See,* Helow and Khattab, [1996] Acta Microbiol. Immunol. Hung. 43:289-299), *B. ovatus* mannanase *(See,* Gherardini etal., [1987] J. Bacterial. 169:2038-2043), *B. ruminicola* mannanase *(See,* Matsushita et al., [1991] J. Bacterial. 173:6919-6926), *C*. *cellulovorans* mannanase *(See,* Sunna et al., [2000] Appl. Environ. Microbiol. 66:664-670), *C*. *saccharolyticus* mannanase *(See,* Morris et al., [1995] Appl. Environ. Microbiol. 61: 2262-2269), *C*. *saccharolyticum* mannanase *(See,* Bicho et al., [1991] Appl. Microbiol. Biotechnol. 36:337-343), *C. fimi* mannanase *(See,* Stoll etal., [1999] Appl. Environ. Microbiol. 65(6):2598-2605), *C*. *butyricum*/*beijerinckii* mannanase *(See,* Nakajima and Matsuura, [1997] Biosci. Biotechnol. Biochem. 61:1739-1742), *C*. *cellulolyticum* mannanase *(See,* Perret et al., [2004] Biotechnol. Appl. Biochem. 40:255-259), *C*. *tertium* mannanase *(See,* Kataoka and Tokiwa, [1998] J. Appl. Microbiol. 84:357-367), *C*. *thermocellum* mannanase *(See,* Halstead et al., [1999] Microbiol. 145:3101-3108), *D. thermophilum* mannanase *(See,* Gibbs et al., [1999] Curr. Microbiol. 39(6):351-357), *Flavobacterium sp.* mannanase *(See,* Zakaria et al., [1998] Biosci. Biotechnol. Biochem. 62:655-660), *G. pulmonata* mannanase *(See,* Charrier and Rouland, [2001] J. Expt. Zool. 290: 125-135), *L. brevicula* mannanase *(See,* Yamamura et al., [1996] Biosci. Biotechnol. Biochem. 60:674-676), *L. esculentum* mannanase *(See,* Filichkin et al., [2000] Plant Physiol. 134:1080-1087), *P. curdlanolyticus* mannanase *(See,* Pason and Ratanakhanokchai, [2006] Appl. Environ. Microbiol. 72:2483-2490), *P. polymyxa* mannanase *(See,* Han et al., [2006] Appl. Microbiol Biotechnol. 73(3):618-630), *P. chrysosporium* mannanase *(See,* Wymelenberg et al., [2005] J. Biotechnol. 118:17-34), *Piromyces sp.* mannanase *(See,* Fanutti et al., [1995] J. Biol. Chem. 270(49):29314-29322), *P. insulars* mannanase *(See,* Yamamura etal., [1993] Biosci. Biotechnol. Biochem. 7:1316-1319), *P. fluorescens* subsp. cellulosa mannanase *(See,* Braithwaite et al., [1995] Biochem J. 305:1005-1010), *R. marinus* mannanase *(See,* Politz et al., [2000] Appl. Microbiol. Biotechnol. 53 (6):715-721), *S.rolfsii* mannanase *(See,* Sachslehner et al., [2000] J. Biotechnol. 80:127-134), *S. galbus* mannanase *(See,* Kansoh and Nagieb, [2004] Anton. van. Leeuwenhoek. 85:103-114), *S. lividans* mannanase *(See,* Arcand et al., [1993] J.Biochem. 290:857-863), *T. Polysaccharolyticum* mannanase *(See,* Cann et al., [1999] J. Bacterial. 181:1643-1651), *T. jusca* mannanase *(See,* Hilge etal., [1998] Structure 6:1433-1444), *T. maritima* mannanase *(See,* Parker et al., [2001] Biotechnol. Bioeng. 75(3):322-333), *T. neapolitana* mannanase *(See,* Duffaud et al., [1997] Appl. Environ. Microbiol. 63:169-177), *T. harzianum* strain T4 mannanase *(See,* Franco et al., [2004] Biotechnol Appl. Biochem. 40:255-259), *T. reesei* mannanase *(See,* Stalbrand et al., [1993] J. Biotechnol. 29:229-242), and *Vibrio sp.* mannanase *(See,* Tamaru et al., [1997] J. Ferment. Bioeng. 83:201-205).

Exemplary commercially available mannanases include, but are not limited to endo-β-mannanases such as HEMICELL^{®} (Chemgen); GAMANASE^{®} and MANNAWAY^{®}, (Novozymes A/S, Denmark); EFFECTENZ^{™} M 1000, PREFERENZ^{®} M 100, PURABRITE^{™} and MANNASTAR^{™} (DuPont); and PYROLASE^{®} 160 and PYROLASE^{®} 200 (Diversa).

In other embodiments, the composition described herein comprises one or more mannanase variant described herein and one or more additional enzyme. The one or more additional enzyme is selected from acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, additional mannanases, metalloproteases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, and any combination or mixture thereof. Some embodiments are directed to a combination of enzymes (i.e., a "cocktail") comprising conventional enzymes like amylase, protease, lipase, cutinase and/or cellulase in conjunction with one or more mannanase variant described herein and/or one or more additional mannanase.

In some embodiments, the cleaning compositions described herein further comprise a protease. In some embodiments the composition comprises from about 0.00001% to about 10% protease by weight of the composition. In another embodiment, the cleaning composition comprises from about 0.0001% to about 10%, about 0.001% to about 5%, about 0.001% to about 2%, or about 0.005% to about 0.5% protease by weight of the composition.

In one embodiment, the protease is a serine protease. Suitable proteases include those of animal, vegetable or microbial origin. In some embodiments, the protease is a microbial protease. In other embodiments, the protease is a chemically or genetically modified mutant. In another embodiment, the protease is an alkaline microbial protease or a trypsin-like protease. Exemplary alkaline proteases include subtilisins derived from, for example, *Bacillus* (e.g., subtilisin, *lentus, amyloliquefaciens,* subtilisin Carlsberg, subtilisin 309, subtilisin 147 and subtilisin 168). Exemplary additional proteases include but are not limited to those described in WO9221760, WO9523221, WO2008010925, WO09149200, WO09149144, WO09149145, WO 10056640, WO10056653, WO20100566356, WO11072099, WO201113022, WO11140364, WO 12151534, WO2015038792, WO2015089447, WO2015089441, WO2015/143360, WO2016 061438, WO2016069548, WO2016069544, WO2016069557, WO2016069563, WO2016 069569, WO2016069552, WO2016145428, US Publ. No. 20080090747, US5801039, US 5340735, US5500364, US5855625, RE34606, US5955340, US5700676, US6312936, US 6482628, US8530219, US Provisional Appl Nos. 62/331282, 62/332417, 62/343618, and 62/351649, and PCT Appl Nos. PCT/US16/32514 and PCT/US2016/038245, as well as metalloproteases described in WO1999014341, WO1999033960, WO1999014342, WO1999 034003, WO2007044993, WO2009058303, WO2009058661, WO2014071410, WO2014 194032, WO2014194034, WO2014194054, and WO2014 194117. Exemplary proteases include, but are not limited to trypsin (e.g., of porcine or bovine origin) and the *Fusarium* protease described in WO8906270. Exemplary commercial proteases include, but are not limited to MAXATASE^{®}, MAXACAL^{™}, MAXAPEM^{™}, OPTICLEAN^{®}, OPTIMASE^{®}, PROPERASE^{®}, PURAFECT^{®}, PURAFECT^{®} OXP, PURAMAX^{™}, EXCELLASE^{™}, PREFERENZ^{™} proteases (e.g. P100, P110, P280), EFFECTENZ^{™} proteases (e.g. P1000, P1050, P2000), EXCELLENZ^{™} proteases (e.g. P1000), ULTIMASE^{®}, and PURAFAST^{™} (DuPont); ALCALASE^{®}, ALCALASE^{®} ULTRA, BLAZE^{®}, BLAZE^{®} EVITY^{®}, BLAZE^{®} EVITY^{®} 16L, CORONASE^{®}, SAVINASE^{®}, SAVINASE^{®} ULTRA, SAVINASE^{®} EVITY^{®}, SAVINASE^{®} EVERIS^{®}, PRIMASE^{®}, DURAZYM^{™}, POLARZYME^{®}, OVOZYME^{®}, KANNASE^{®}, LIQUANASE^{®}, LIQUANASE EVERIS^{®}, NEUTRASE^{®}, PROGRESS UNO^{®}, RELASE^{®} and ESPERASE^{®} (Novozymes); BLAP^{™} and BLAP^{™} variants (Henkel); LAVERGY^{™} PRO 104 L (BASF), and KAP (*B. alkalophilus* subtilisin (Kao)).

In some embodiments, the cleaning compositions described herein further comprise a suitable amylase. In one embodiment, the composition comprises from about 0.00001% to about 10%, about 0.0001% to about 10%, about 0.001% to about 5%, about 0.001% to about 2%, or about 0.005% to about 0.5% amylase by weight of the composition. Any amylase (e.g., alpha and/or beta) suitable for use in alkaline solutions may be useful to include in such composition. An exemplary amylase can be a chemically or genetically modified mutant. Exemplary commercial amylases include, but are not limited amylases described in GB1296839, WO91 00353, WO9402597, WO94183314, WO9510603, WO9526397, WO9535382, WO9605295, WO9623873, WO9623874, WO9630481, WO9710342, WO9741213, WO9743424, WO98 13481, WO9826078, WO9902702, WO9909183, WO9919467, WO9923211, WO9929876, WO9942567, WO9943793, WO9943794, WO9946399, WO0029560, WO0060058, WO00 60059, WO0060060, WO0114532, WO0134784, WO0164852, WO0166712, WO0188107, WO0196537, WO02092797, WO0210355, WO0231124, WO2004055178, WO2004113551, WO2005001064, WO2005003311, WO2005018336, WO 2005019443, WO2005066338, WO 2006002643, WO2006012899, WO2006012902, WO2006 031554, WO2006063594, WO2006 066594, WO2006066596, WO2006136161, WO2008 000825, WO2008088493, WO2008 092919, WO2008101894, WO2008112459, WO2009 061380, WO2009061381, WO2009 100102, WO2009140504, WO2009149419, WO2010 059413, WO2010088447, WO2010 091221, WO2010104675, WO2010 115021, WO10115028, WO2010117511, WO2011076123, WO2011076897, WO2011080352, WO2011080353, WO2011080354, WO2011082425, WO 2011082429, WO2011087836, WO2011098531, WO2013063460, WO2013184577, WO2014 099523, WO2014164777, and WO2015077126. Exemplary commercial amylases include, but are not limited to AMPLIFY^{®}, AMPLIFY PRIME^{®}, BAN^{™}, DURAMYL^{®}, TERMAMYL^{®}, TERMAMYL^{®} ULTRA, FUNGAMYL^{®}, STAINZYME^{®}, STAINZYME PLUS^{®}, STAINZYME ULTRA^{®}, and STAINZYME EVITY^{®}(Novozymes); EFFECTENZ^{™} S 1000, POWERASE^{™}, PREFERENZ^{™} S 100, PREFERENZ^{™} S 110, EXCELLENZ^{™} S 2000, RAPIDASE^{®} and MAXAMYL^{®} P (DuPont).

In some embodiments, the cleaning compositions described herein further comprise a suitable pectin degrading enzyme. As used herein, "pectin degrading enzyme(s)" encompass arabinanase (EC 3.2.1.99), galactanases (EC 3.2.1.89), polygalacturonase (EC 3.2.1.15) exo-polygalacturonase (EC 3.2.1.67), exo-poly-alpha-galacturonosidase (EC 3.2.1.82), pectin lyase (EC 4.2.2.10), pectin esterase (EC 3.1.1.11), pectate lyase (EC 4.2.2.2), exo-polygalacturonate lyase (EC 4.2.2.9) and hemicellulases such as endo-1,3-β-xylosidase (EC 3.2.1.32), xylan-1,4-β-xylosidase (EC 3.2.1.37) and α-L-arabinofuranosidase (EC 3.2.1.55). Pectin degrading enzymes are natural mixtures of the above mentioned enzymatic activities. Pectin enzymes therefore include the pectin methylesterases which hydrolyse the pectin methyl ester linkages, polygalacturonases which cleave the glycosidic bonds between galacturonic acid molecules, and the pectin transeliminases or lyases which act on the pectic acids to bring about non-hydrolytic cleavage of α-1,4 glycosidic linkages to form unsaturated derivatives of galacturonic acid.

Suitable pectin degrading enzymes include those of plant, fungal, or microbial origin. In some embodiments, chemically or genetically modified mutants are included. In some embodiments, the pectin degrading enzymes are alkaline pectin degrading enzymes, *i.e.,* enzymes having an enzymatic activity of at least 10%, at least 25%, or at least 40% of their maximum activity at a pH of from about 7.0 to about 12. In certain other embodiments, the pectin degrading enzymes are enzymes having their maximum activity at a pH of from about 7.0 to about 12. Alkaline pectin degrading enzymes are produced by alkalophilic microorganisms *e.g.,* bacterial, fungal, and yeast microorganisms such as *Bacillus* species. In some embodiments, the microorganisms are *B. firmus, B. circulans,* and *B. subtilis* as described in JP 56131376 and JP 56068393. Alkaline pectin decomposing enzymes may include but are not limited to galacturan-1,4-α-galacturonidase (EC 3.2.1.67), poly-galacturonase activities (EC 3.2.1.15, pectin esterase (EC 3.1.1.11), pectate lyase (EC 4.2.2.2) and their iso enzymes. Alkaline pectin decomposing enzymes can be produced by the Erwinia species. In some embodiments, the alkaline pectin decomposing enzymes are produced by *E.chrysanthemi, E.carotovora, E.amylovora, E.herbicola,* and *E.dissolvens* as described in JP 59066588, JP 63042988, and in World J. Microbiol. Biotechnol. (8, 2, 115-120) 1992. In certain other embodiments, the alkaline pectin enzymes are produced by *Bacillus* species as disclosed in JP 73006557 and Agr. Biol. Chem. (1972), 36 (2) 285-93. In some embodiments, the cleaning compositions described herein further comprise about 0.00001% to about 10%, about 0.0001% to about 10%, about 0.001% to about 5%, about 0.001% to about 2%, or about 0.005% to about 0.5% of pectin degrading enzyme by weight of the composition.

In some other embodiments, the cleaning compositions described herein further comprise a suitable xyloglucanase. Suitable xyloglucanases include, but are not limited to those of plant, fungal, or bacterial origin. Chemically or genetically modified mutants are included in some embodiments. As used herein, "xyloglucanase(s)" encompass the family of enzymes described by Vincken and Voragen at Wageningen University [Vincken et al (1994) Plant Physiol., 104, 99-107] and are able to degrade xyloglucans as described in Hayashi et al (1989) Annu. Rev. Plant. Physiol. Plant Mol. Biol., 40, 139-168. Vincken et al demonstrated the removal of xyloglucan coating from cellulose of the isolated apple cell wall by a xyloglucanase purified from *Trichoderma viride* (endo-IV-glucanase). This enzyme enhances the enzymatic degradation of cell wall-embedded cellulose and work in synergy with pectic enzymes. Rapidase LIQ+ from DSM contains a xyloglucanase activity. In some embodiments, the cleaning compositions described herein further comprise from about 0.00001% to about 10%, about 0.0001% to about 10%, about 0.001% to about 5%, about 0.001% to about 2%, or about 0.005% to about 0.5% xyloglucanase by weight of the composition. In certain other embodiments, xyloglucanases for specific applications are alkaline xyloglucanases, *i.e.,* enzymes having an enzymatic activity of at least 10%, at least 25%, or at least 40% of its maximum activity at a pH ranging from 7 to 12. In certain other embodiments, the xyloglucanases are enzymes having a maximum activity at a pH of from about 7.0 to about 12.

In some further embodiments, the detergent compositions described herein further comprise a suitable cellulase. In one embodiment, the composition comprises from about 0.00001 % to about 10%, 0.0001% to about 10%, about 0.001% to about 5%, about 0.001% to about 2%, or about 0.005% to about 0.5% cellulase by weight of the composition. Any suitable cellulase may find use in a composition described herein. An exemplary cellulase can be a chemically or genetically modified mutant. Exemplary cellulases include but are not limited, to those of bacterial or fungal origin, such as, for example, is described in WO2005054475, WO2005056787, US 7,449,318, US 7,833,773, US 4,435,307; EP 0495257; and US Provisional Appl. No. 62/296,678. Exemplary commercial cellulases include, but are not limited to CELLUCLEAN^{®}, CELLUZYME^{®}, CAREZYME^{®}, ENDOLASE^{®}, RENOZYME^{®}, and CAREZYME^{®} PREMIUM (Novozymes); REVITALENZ^{™} 100, REVITALENZ^{™} 200/220, and REVITALENZ^{®} 2000 (DuPont); and KAC-500(B)^{™} (Kao Corporation). In some embodiments, cellulases are incorporated as portions or fragments of mature wild-type or variant cellulases, wherein a portion of the N-terminus is deleted *(see, e.g.,* US 5,874,276).

In still further embodiments, the detergent compositions described herein further comprise a suitable lipase. In some embodiments, the composition comprises from about 0.00001 % to about 10%, about 0.0001 % to about 10%, about 0.001% to about 5%, about 0.001% to about 2%, or about 0.005% to about 0.5% lipase by weight composition. An exemplary lipase can be a chemically or genetically modified mutant. Exemplary lipases include, but are not limited to, e.g., those of bacterial or fungal origin, such as, e.g., *H. lanuginosa* lipase *(see, e.g.,* EP 258068 and EP 305216), *T. lanuginosus* lipase *(see, e.g.,* WO 2014/059360 and WO2015/010009), *Rhizomucor miehei* lipase *(see, e.g.,* EP 238023), *Candida* lipase, such as C. *antarctica* lipase (e.g., C. *antarctica* lipase A or B) *(see, e.g.,* EP 214761), *Pseudomonas* lipases such as *P. alcaligenes* and *P. pseudoalcaligenes* lipase *(see, e.g.,* EP 218272), *P. cepacia* lipase *(see, e.g.,* EP 331376), *P. stutzeri* lipase *(see, e.g.,* GB 1,372,034), *P*. *fluorescens* lipase, *Bacillus* lipase (e.g., *B. subtilis* lipase (Dartois et al., Biochem. Biophys. Acta 1131:253-260 (1993)), *B. stearothermophilus* lipase *(see, e.g.,* JP 64/744992), and *B. pumilus* lipase *(see, e.g.,* WO 91/16422)). Exemplary cloned lipases include, but are not limited to *Penicillium camembertii* lipase *(See,* Yamaguchi et al., Gene 103:61-67 (1991)), *Geotricum candidum* lipase *(See,* Schimada et al., J. Biochem., 106:383-388 (1989)), and various *Rhizopus* lipases, such as, *R. delemar* lipase *(See,* Hass et al., Gene 109:117-113 (1991)), *R. niveus* lipase (Kugimiya et al., Biosci. Biotech. Biochem. 56:716-719 (1992)) and *R*. *oryzae* lipase. Other lipolytic enzymes, such as cutinases, may also find use in one or more composition described herein, including, but not limited to, e.g., cutinase derived from *Pseudomonas mendocina* (*see,* WO 88/09367) and/or *Fusarium solani pisi* (*see,* WO90/09446). Exemplary commercial lipases include, but are not limited to M1 LIPASE^{™}, LUMA FAST^{™}, and LIPOMAX^{™} (DuPont); LIPEX^{®}, LIPOCLEAN^{®}, LIPOLASE^{®} and LIPOLASE^{®} ULTRA (Novozymes); and LIPASE P^{™} (Amano Pharmaceutical Co. Ltd).

In some embodiments, cleaning compositions described herein further comprise peroxidases in combination with hydrogen peroxide or a source thereof *(e.g.,* a percarbonate, perborate or persulfate). In some alternative embodiments, oxidases are used in combination with oxygen. Both types of enzymes are used for "solution bleaching" *(i.e.,* to prevent transfer of a textile dye from a dyed fabric to another fabric when the fabrics are washed together in a wash liquor), preferably together with an enhancing agent *(See, e.g.,* WO94/12621 and WO95/01426). Suitable peroxidases/oxidases include, but are not limited to those of plant, bacterial or fungal origin. Chemically or genetically modified mutants are included in some embodiments. In some embodiments, the cleaning compositions of the present disclosure further comprise from about 0.00001% to about 10%, about 0.0001% to about 10%, about 0.001% to about 5%, about 0.001% to about 2%, about 0.005% to about 0.5% of peroxidase and/or oxidase by weight of the composition.

In some embodiments, cleaning compositions described herein further comprise additional enzymes, including but not limited to perhydrolases *(See, e.g.,* WO 05/056782). Some embodiments are directed to mixtures of one or more above mentioned protease, amylase, lipase, mannanase, and/or cellulase.

Some embodiments are directed to cleaning compositions such as, for example, those described in US 6,605,458. In some embodiments, the cleaning compositions described herein are compact granular fabric cleaning compositions, while in other embodiments the composition is a granular fabric cleaning composition useful in the laundering of colored fabrics. In further embodiments, the composition is a granular fabric cleaning composition which provides softening through the wash capacity, and in additional embodiments the composition is a heavy duty liquid (HDL) fabric cleaning composition. In other embodiments, the cleaning compositions described herein are fabric cleaning compositions such as, for example, those described in US 6,610,642 and 6,376,450. In an alternative embodiment, the cleaning compositions described herein are suitable hard surface cleaning compositions. Suitable hard surface cleaning compositions include, for example, those described in US 6,610,642; 6,376,450; and 6,376,450. In yet further embodiments, the cleaning compositions described herein are dishwashing compositions. In some further embodiments, the compositions described herein are oral care compositions such as, for example, those described in US 6,376,450 and 6,605,458. The formulations and descriptions of the compounds and cleaning adjunct materials contained in the aforementioned US 6,376,450; 6,605,458; and 6,610,642 find use with a polypeptide of the present invention.

In still further embodiments, the cleaning compositions described herein are fabric softening compositions such as, for example, those described in GB 400898, GB 514 276, EP0011340, EP0026528, EP0242919, EP0299575, EP0313146, and US 5,019,292.

The cleaning compositions described herein can be formulated into any suitable form and prepared by any process chosen by the formulator, non-limiting examples of which are described in US 5,879,584; 5,691,297; 5,574,005; 5,569,645; 5,565,422; 5,516,448; 5,489,392; and 5,486,303. When a low pH cleaning composition is desired, the pH of such composition is adjusted via the addition of a material such as monoethanolamine or an acidic material such as HCl.

In some embodiments, the cleaning compositions described herein are provided in unit dose form, including tablets, capsules, sachets, pouches, sheets, and multi-compartment pouches. In some embodiments, the unit dose format is designed to provide controlled release of the ingredients within a multi-compartment pouch (or other unit dose format). Suitable unit dose and controlled release formats are known in the art (*See e.g.,* EP2100949, EP2100947, WO02/102955, WO04/111178, WO2013/165725, and US 4,765,916 and 4,972,017). In some embodiments, the unit dose form is provided by tablets wrapped with a water-soluble film or water-soluble pouches.

In some embodiments, the cleaning compositions described herein further comprise at least one chelating agent. Suitable chelating agents may include, but are not limited to copper, iron, and/or manganese chelating agents, and mixtures thereof. In embodiments in which at least one chelating agent is used, the cleaning compositions of the present disclosure comprise from about 0.1% to about 15% or even from about 3.0% to about 10% chelating agent by weight of the cleaning composition.

In some still further embodiments, the cleaning compositions described herein further comprise at least one deposition aid. Suitable deposition aids include, but are not limited to, polyethylene glycol, polypropylene glycol, polycarboxylate, soil release polymers such as polyterephthalic acid, clays such as kaolinite, montmorillonite, attapulgite, illite, bentonite, halloysite, and mixtures thereof.

In some embodiments, the cleaning compositions described herein further comprise at least one anti-redeposition agent. In some embodiments, the anti-redeposition agent is a non-ionic surfactant, such as, for example, described in EP2100949. In some automatic dishwashing embodiments, non-ionic surfactants are used as surface modifiers, in particular for sheeting, to avoid filming and spotting and to improve shine.

In some embodiments, the cleaning compositions described herein further comprise one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones, and polyvinylimidazoles, or mixtures thereof. In some embodiments, the cleaning compositions described herein comprise from about 0.0001% to about 10%, from about 0.01% to about 5%, or even from about 0.1% to about 3% dye transfer inhibiting agent by weight of the cleaning composition.

In some embodiments, the cleaning compositions described herein further comprise one or more silicates. In some such embodiments, sodium silicates (e.g., sodium disilicate, sodium metasilicate, and crystalline phyllosilicates) find use. In some embodiments, the cleaning compositions described herein comprise from about 1% to about 20% or from about 5% to about 15% silicate by weight of the composition.

In yet further embodiments, the cleaning compositions described herein further comprise one or more dispersant. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning compositions are stabilized by any suitable technique. In some embodiments, the enzymes employed herein are stabilized by the presence of water-soluble sources of calcium and/or magnesium ions in the finished compositions. In some embodiments, the enzyme stabilizers include oligosaccharides, polysaccharides, and inorganic divalent metal salts, including alkaline earth metals, such as calcium salts. It is contemplated that various techniques for enzyme stabilization will find use in the present disclosure. For example, in some embodiments, the enzymes employed herein are stabilized by the presence of water-soluble sources of zinc (II), calcium (II), and/or magnesium (II) ions in the finished compositions, as well as other metal ions (e.g., barium (II), scandium (II), iron (II), manganese (II), aluminum (III), tin (II), cobalt (II), copper (II), nickel (II), and oxovanadium (IV)). Chlorides and sulfates also find use in some embodiments. Examples of suitable oligosaccharides and polysaccharides *(e.g.,* dextrins) are known in the art (*See, e.g.,* WO07/145964). In some embodiments, reversible protease inhibitors, such as boron-containing compounds (e.g., borate, 4-formyl phenyl boronic acid) and/or a tripeptide aldehyde find use to further improve stability.

In some embodiments, the cleaning compositions described herein further comprise one or more bleach, bleach activator, and/or bleach catalyst. In some embodiments, the cleaning compositions described herein comprise inorganic and/or organic bleaching compound(s). Inorganic bleaches may include, but are not limited to perhydrate salts (e.g., perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Suitable salts include, for example, those described in EP2100949. Bleach activators are typically organic peracid precursors that enhance the bleaching action in the course of cleaning at temperatures of 60°C and below. Bleach activators suitable for use herein include compounds which, under perhydrolysis conditions, give aliphatic peroxycarboxylic acids having preferably from about 1 to about 10 carbon atoms, in particular from about 2 to about 4 carbon atoms, and/or optionally substituted perbenzoic acid. Suitable bleach activators include, for example, those described in EP2100949. Bleach catalysts typically include, for example, manganese triazacyclononane and related complexes, and cobalt, copper, manganese, and iron complexes, as well as those described in US 4,246,612; 5,227,084; 4,810,410; and WO99/06521and EP2100949.

In some embodiments, the cleaning compositions described herein further comprise one or more catalytic metal complex. In some embodiments, a metal-containing bleach catalyst finds use. In other embodiments, the metal bleach catalyst comprises a catalyst system comprising a transition metal cation of defined bleach catalytic activity (e.g., copper, iron, titanium, ruthenium, tungsten, molybdenum, or manganese cations), an auxiliary metal cation having little or no bleach catalytic activity *(e.g.,* zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used *(See, e.g.,* US 4,430,243). In some embodiments, the cleaning compositions described herein are catalyzed by means of a manganese compound. Such compounds and levels of use are well known in the art *(See, e.g.,* US 5,576,282). In additional embodiments, cobalt bleach catalysts find use in the cleaning compositions described herein. Various cobalt bleach catalysts are known in the art *(See, e.g.,* US 5,597,936 and 5,595,967) and are readily prepared by known procedures.

In some additional embodiments, the cleaning compositions described herein further comprise a transition metal complex of a macropolycyclic rigid ligand (MRL). As a practical matter, and not by way of limitation, in some embodiments, the compositions and cleaning processes provided herein are adjusted to provide on the order of at least one part per hundred million of the active MRL species in the aqueous washing medium, and in other embodiments, provide from about 0.005 ppm to about 25 ppm, from about 0.05 ppm to about 10 ppm, or from about 0.1 ppm to about 5 ppm of the MRL in the wash liquor.

In some embodiments, the transition-metal in the instant transition-metal bleach catalyst include, but are not limited to manganese, iron, and chromium. In other embodiments, MRI,s include, but are not limited to special ultra-rigid ligands that are cross-bridged (e.g., 5,12-diethyl-1,5,8,12-tetraazabicyclo[6.6.2] hexadecane). Suitable transition metal MRLs are readily prepared by known procedures *(See, e.g.,* WO 2000/32601 and US 6,225,464).

In some embodiments, the cleaning compositions described herein further comprise a metal care agent. Metal care agents are used to prevent and/or reduce tarnishing, corrosion, and/or oxidation of metals, including aluminum, stainless steel, and non-ferrous metals (e.g., silver and copper). Suitable metal care agents include those described in EP2100949, WO94/26860, and WO94/26859). In some embodiments, the metal care agent is a zinc salt. In some further embodiments, the cleaning compositions described herein comprise from about 0.1% to about 5% by weight of one or more metal care agent.

The cleaning compositions described herein can be used to clean a surface, dishware, or fabric. Typically, at least a portion of the surface, dishware, or fabric is contacted with at least one (i) variant or recombinant polypeptide or active fragment thereof described herein, or (ii) at least one cleaning composition described herein, and then the surface, dishware, or fabric is optionally washed and/or rinsed. For purposes of the present disclosure, "washing" includes but is not limited to, scrubbing and mechanical agitation. In some embodiments, the cleaning compositions are typically employed at concentrations of from about 500 ppm to about 15,000 ppm in solution. When the wash solvent is water, the water temperature typically ranges from about 5°C to about 90°C and, when fabric is involved, the water to fabric mass ratio is typically from about 1:1 to about 30:1.

Some embodiments are directed to a method of cleaning comprising contacting an effective amount of (i) a mannanase variant as set forth in the claims, or (ii) a cleaning composition as set forth in the claims with an item or surface comprising a soil or stain comprising mannan to hydrolyze the mannan contained in the soil or stain.

Disclosed herein, one or more mannanase variant or recombinant polypeptide or active fragment thereof described herein is used to prevent, reduce and/or remove a biofilm on one or more item selected from a textile and fabric. One or more mannanase variant or recombinant polypeptide or active fragment thereof described herein hydrolyzes polysaccharide chains containing mannose units, including, but not limited to, mannans, galactomannans, and glucomannans, making such polypeptides particularly useful for performing mannan hydrolysis reactions involving polysaccharide substrates containing 1,4-β-D-mannosidic linkages.

In general terms, a donor molecule is incubated in the presence of a mannanase variant or recombinant polypeptide or active fragment thereof described herein under conditions suitable for performing a mannan hydrolysis reaction, followed by, optionally, isolating a product from the reaction. Alternatively, in the context of a foodstuff, the product may become a component of the foodstuff without isolation. In certain embodiments, the donor molecule is a polysaccharide chain comprising mannose units, including but not limited to mannans, glucomannans, galactomannans, and galactoglucomannans.

Disclosed herein, one or more mannanase variants or recombinant polypeptide or active fragment thereof described herein is used in a process for extracting palm kernel oil. Also disclosed is a process for extracting palm kernel oil from palm kernels or a palm kernel meal, comprising providing palm kernels and/or palm kernel meal and treating said seeds or cake with one or more mannanase variant or recombinant polypeptide or active fragment thereof described herein.

Disclosed herein, a composition comprising a mannanase variant or recombinant polypeptide or active fragment thereof described herein is used to process and/or manufacture animal feed or food for humans. Also disclosed, a mannanase variant or recombinant polypeptide or active fragment thereof described herein can be an additive to feed for non-human animals. Also disclosed, a mannanase variant or recombinant polypeptide or active fragment thereof described herein can be useful for human food, such as, for example, as an additive to human food.

Several nutritional factors can limit the amount of inexpensive plant material that can be used to prepare animal feed and food for humans. For example, plant material containing oligomannans such as mannan, galactomannan, glucomannan and galactoglucomannan can reduce an animal's ability to digest and absorb nutritional compounds such as minerals, vitamins, sugars, and fats. These negative effects are in particular due to the high viscosity of the mannan-containing polymers and to the ability of the mannan-containing polymers to absorb nutritional compounds. These effects can be reduced by including an enzyme in the feed that degrades the mannan-containing polymers, such as, an endo-β-mannanase enzyme described herein, thereby enabling a higher proportion of mannan-containing polymers typically found in inexpensive plant material to be included in the feed, which ultimately reduces the cost of the feed. Additionally, a mannanase variant or recombinant polypeptide or active fragment thereof described herein can break down the mannan-containing polymers into simpler sugars, which can be more readily assimilated to provide additional energy.

Disclosed herein, animal feed containing plant material is incubated in the presence of a mannanase variant or recombinant polypeptide or active fragment thereof described herein under conditions suitable for breaking down mannan-containing polymers.

Also disclosed herein is a bread improver composition comprises a mannanase variant or recombinant polypeptide or active fragment thereof described herein, optionally in combination with a source of mannan or glucomannan or galactomannan, and further optionally in combination with one or more other enzymes.

The term "non-human animal" includes all non-ruminant and ruminant animals. In a particular embodiment, the non-ruminant animal is selected from the group consisting of, but is not limited to, horses and monogastric animals such as, but not limited to, pigs, poultry, swine and fish. In further embodiments, the pig may be, but is not limited to, a piglet, a growing pig, and a sow; the poultry may be, but is not limited to, a turkey, a duck and a chicken including, but not limited to, a broiler chick and a layer; and fish may be, but is not limited to salmon, trout, tilapia, catfish and carps; and crustaceans including but not limited to shrimps and prawns. In a further embodiment, the ruminant animal is selected from the group consisting of, but is not limited to, cattle, young calves, goats, sheep, giraffes, bison, moose, elk, yaks, water buffalo, deer, camels, alpacas, llamas, antelope, pronghorn, and nilgai.

Disclosed herein, a mannanase variant or recombinant polypeptide or active fragment thereof described herein is used to pretreat feed instead of as a feed additive. Also disclosed, a mannanase variant or recombinant polypeptide or active fragment thereof described herein is added to, or used to pretreat, feed for weanling pigs, nursery pigs, piglets, fattening pigs, growing pigs, finishing pigs, laying hens, broiler chicks, and turkeys.

Disclosed herein, a mannanase variant or recombinant polypeptide or active fragment thereof described herein is added to, or used to pretreat, feed from plant material such as palm kernel, coconut, konjac, locust bean gum, gum guar, soy beans, barley, oats, flax, wheat, corn, linseed, citrus pulp, cottonseed, groundnut, rapeseed, sunflower, peas, and lupines.

A mannanase variant or recombinant polypeptide or active fragment thereof described herein is thermostable, and as a result, a mannanase variant or recombinant polypeptide or active fragment thereof described herein can be used in processes of producing pelleted feed in which heat is applied to the feed mixture before the pelleting step. Disclosed herein, a mannanase variant or recombinant polypeptide or active fragment thereof described herein is added to the other feed ingredients either in advance of the pelleting step or after the pelleting step (i.e., to the already formed feed pellets).

Also disclosed herein, food processing or feed supplement compositions that contain a mannanase variant or recombinant polypeptide or active fragment thereof described herein may optionally further contain other substituents selected from coloring agents, aroma compounds, stabilizers, vitamins, minerals, and other feed or food enhancing enzymes. This applies in particular to the so-called pre-mixes.

A food additive may be combined in an appropriate amount with other food components, such as, for example, a cereal or plant protein to form a processed food product.

A animal feed composition and/or animal feed additive composition and/or pet food may comprise a polypeptide described herein.

Disclosed herein is a method for preparing an animal feed composition and/or animal feed additive composition and/or pet food comprising mixing a mannanase variant or recombinant polypeptide or active fragment thereof described herein with one or more animal feed ingredients and/or animal feed additive ingredients and/or pet food ingredients.

Disclosed herein is the use of a mannanase variant or recombinant polypeptide or active fragment thereof described herein to prepare an animal feed composition and/or animal feed additive composition and/or pet food. The phrase "pet food" means food for a household animal such as, but not limited to, dogs; cats; gerbils; hamsters; chinchillas; fancy rats; guinea pigs; avian pets, such as canaries, parakeets, and parrots; reptile pets, such as turtles, lizards and snakes; and aquatic pets, such as tropical fish and frogs.

The terms animal feed composition, feedstuff and fodder are used interchangeably and may comprise one or more feed materials selected from the group comprising a) cereals, such as small grains (e.g., wheat, barley, rye, oats and combinations thereof) and/or large grains such as maize or sorghum; b) by-products from cereals, such as corn gluten meal, Distillers Dried Grain Solubles (DDGS) (particularly corn based Distillers Dried Grain Solubles (cDDGS)), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, and sesame; d) oils and fats obtained from vegetable and animal sources; and e) minerals and vitamins.

The food composition or additive may be liquid or solid.

A food composition disclosed herein can be a beverage, including, but not limited to, a fermented beverage such as beer and wine.

In the context of the present disclosure, the term "fermented beverage" is meant to comprise any beverage produced by a method comprising a fermentation process, such as a microbial fermentation, such as a bacterial and/or yeast fermentation.

A fermented beverage can be beer. The term "beer" is meant to comprise any fermented wort produced by fermentation/brewing of a starch-containing plant material. Often, beer is produced from malt or adjunct, or any combination of malt and adjunct as the starch-containing plant material. As used herein the term "malt" is understood as any malted cereal grain, such as malted barley or wheat.

As used herein the term "adjunct" refers to any starch and/or sugar containing plant material which is not malt, such as barley or wheat malt. Examples of adjuncts include, for example, common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, cassava and syrups, such as corn syrup, sugar cane syrup, inverted sugar syrup, barley and/or wheat syrups, and the like may be used as a source of starch.

As used herein, the term "mash" refers to an aqueous slurry of any starch and/or sugar containing plant material such as grist, e. g. comprising crushed barley malt, crushed barley, and/or other adjunct or a combination hereof, mixed with water, later to be separated into wort and spent grains.

As used herein, the term "wort" refers to the unfermented liquor run-off following extracting the grist during mashing.

Also disclosed herein is a method of preparing a fermented beverage such as beer comprising mixing a mannanase variant or recombinant polypeptide or active fragment thereof described herein with a malt and/or adjunct.

Exemplary beers include, but are not limited to full malted beer, beer brewed under the "Reinheitsgebot", ale, IPA, lager, bitter, Happoshu (second beer), third beer, dry beer, near beer, light beer, low alcohol beer, low calorie beer, porter, bock beer, stout, malt liquor, non-alcoholic beer, non-alcoholic malt liquor, as well as alternative cereal and malt beverages such as fruit flavoured malt beverages, e. g. citrus flavoured, such as lemon-, orange-, lime-, or berry-flavoured malt beverages; liquor flavoured malt beverages, e. g. , vodka-, rum-, or tequila-flavoured malt liquor; or coffee flavoured malt beverages, such as caffeine-flavoured malt liquor.

Also disclosed herein is a use of a mannanase variant or recombinant polypeptide or active fragment thereof described herein in the production of a fermented beverage, such as a beer.

Also disclosed herein is a method of providing a fermented beverage comprising the step of contacting a mash and/or wort with a mannanase variant or recombinant polypeptide or active fragment thereof described herein.

Disclosed herein is a method of providing a fermented beverage comprising the steps of: (a) preparing a mash, (b) filtering the mash to obtain a wort, and (c) fermenting the wort to obtain a fermented beverage, such as a beer, wherein a mannanase variant or recombinant polypeptide or active fragment thereof described herein is added to: (i) the mash of step (a) and/or (ii) the wort of step (b) and/or (iii) the wort of step (c).

Disclosed herein, a fermented beverage, such as a beer, is produced or provided by a method comprising the step(s) of (1) contacting a mash and/or a wort with a mannanase variant or recombinant polypeptide or active fragment thereof described herein; and/or (2) (a) preparing a mash, (b) filtering the mash to obtain a wort, and (c) fermenting the wort to obtain a fermented beverage, such as a beer, wherein a mannanase variant or recombinant polypeptide or active fragment thereof described herein is added to: (i) the mash of step (a) and/or (ii) the wort of step (b) and/or (iii) the wort of step (c).

A mannanase variant or recombinant polypeptide or active fragment thereof described herein may also be used for hydrolyzing galactomannans present in liquid coffee extracts. In one aspect, a mannanase variant or recombinant polypeptide or active fragment thereof described herein is used to inhibit gel formation during freeze drying of liquid coffee extracts. The decreased viscosity of the extract reduces the energy consumption during drying. In certain other aspects, a polypeptide of the present inventions is applied in an immobilized form in order to reduce enzyme consumption and avoid contamination of the coffee extract. This use is further disclosed in EP676145.

In general terms the coffee extract is incubated in the presence of a mannanase variant or recombinant polypeptide or active fragment thereof described herein under conditions suitable for hydrolyzing galactomannans present in liquid coffee extract.

Also disclosed herein is a method of preparing baked products comprising addition of a mannanase variant or recombinant polypeptide or active fragment thereof described herein to dough, followed by baking the dough. Examples of baked products are well known to those skilled in the art and include breads, rolls, puff pastries, sweet fermented doughs, buns, cakes, crackers, cookies, biscuits, waffles, wafers, tortillas, breakfast cereals, extruded products, and the like.

A mannanase variant or recombinant polypeptide or active fragment thereof described herein may be added to dough as part of a bread improver composition. Bread improvers are compositions containing a variety of ingredients, which improve dough properties and the quality of bakery products, e.g. bread and cakes. Bread improvers are often added in industrial bakery processes because of their beneficial effects e.g. the dough stability and the bread texture and volume. Bread improvers usually contain fats and oils as well as additives like emulsifiers, enzymes, antioxidants, oxidants, stabilizers and reducing agents. In addition to any of the polypeptides of the present invention, other enzymes which may also be present in the bread improver or which may be otherwise used in conjunction with any of the polypeptides of the present invention include amylases, hemicellulases, amylolytic complexes, lipases, proteases, xylanases, pectinases, pullulanases, nonstarch polysaccharide degrading enzymes and redox enzymes like glucose oxidase, lipoxygenase or ascorbic acid oxidase.

A mannanase variant or recombinant polypeptide or active fragment thereof described herein may be added to dough as part of a bread improver composition which also comprises a glucomannan and/or galactomannan source such as konjac gum, guar gum, locust bean gum (Ceratonia siliqua), copra meal, ivory nut mannan (Phytelephas macrocarpa), seaweed mannan extract, coconut meal, and the cell wall of brewer's yeast (may be dried, or used in the form of brewer's yeast extract). Other acceptable mannan derivatives for use in the current invention include unbranched β-1,4-linked mannan homopolymer and manno-oligosaccharides (mannobiose, mannotriose, mannotetraose and mannopentoase). A mannanase variant or recombinant polypeptide or active fragment thereof described herein can be further used either alone, or in combination with a glucomannan and/or galactomannan and/or galactoglucomannan to improve the dough tolerance; dough flexibility and/or dough stickiness; and/or bread crumb structure, as well as retarding staling of the bread. In another aspect, the mannanase hydrolysates act as soluble prebiotics such as manno-oligosaccharides (MOS) which promote the growth of lactic acid bacteria commonly associated with good health when found at favourable population densities in the colon. In one aspect, the dough to which any polypeptide of the invention is added comprises bran or oat, rice, millet, maize, or legume flour in addition to or instead of pure wheat flour (i.e., is not a pure white flour dough).

A a mannanase variant or recombinant polypeptide or active fragment thereof described herein may be added to milk or any other dairy product to which has also been added a glucomannan and/or galactomannan. Typical glucomannan and/or galactomannan sources are listed above in the bakery aspects, and include guar or konjac gum. The combination of a mannanase variant or recombinant polypeptide or active fragment thereof described herein with a glucomannan and/or galactomannan releases mannanase hydrolysates (mannooligosaccharides) which act as soluble prebiotics by promoting the selective growth and proliferation of probiotic bacteria (especially *Bifidobacteria* and *Lactobacillus* lactic acid bacteria) commonly associated with good health when found at favourable population densities in the large intestine or colon.

Another aspect relates to a method of preparing milk or dairy products comprising addition of a mannanase variant or recombinant polypeptide or active fragment thereof described herein and any glucomannan or galactomannan or galactoglucomannan.

In another aspect, a mannanase variant or recombinant polypeptide or active fragment thereof described herein is used in combination with any glucomannan or galactomannan prior to or following addition to a dairy based foodstuff to produce a dairy based foodstuff comprising prebiotic mannan hydrolysates. In a further aspect, the thusly produced mannooligosacharide-containing dairy product is capable of increasing the population of beneficial human intestinal microflora, and in a yet further aspect the dairy based foodstuff may comprise a mannanase variant or recombinant polypeptide or active fragment thereof described herein together with any source of glucomannan and/or galactomannan and/or galactoglucomannan, and a dose sufficient for inoculation of at least one strain of bacteria (such as *Bifidobacteria* or *Lactobacillus*) known to be of benefit in the human large intestine. In one aspect, the dairy-based foodstuff is a yoghurt or milk drink.

The mannanase variant or recombinant polypeptide or active fragment thereof described herein finds further use in the enzyme aided bleaching of paper pulps such as chemical pulps, semi-chemical pulps, kraft pulps, mechanical pulps, and pulps prepared by the sulfite method. In general terms, paper pulps are incubated with a mannanase variant or recombinant polypeptide or active fragment thereof described herein under conditions suitable for bleaching the paper pulp.

The pulps may be chlorine free pulps bleached with oxygen, ozone, peroxide or peroxyacids. Disclosed herein, a mannanase variant or recombinant polypeptide or active fragment thereof described herein is used in enzyme aided bleaching of pulps produced by modified or continuous pulping methods that exhibit low lignin contents. Also disclosed herein, a mannanase variant or recombinant polypeptide or active fragment thereof described herein is applied alone or preferably in combination with xylanase and/or endoglucanase and/or alpha-galactosidase and/or cellobiohydrolase enzymes.

Galactomannans such as guar gum and locust bean gum are widely used as thickening agents e.g., in food (e.g., ice cream) and print paste for textile printing such as prints on T-shirts. Thus, a mannanase variant or recombinant polypeptide or active fragment thereof described herein also finds use in reducing the thickness or viscosity of mannan-containing substrates. Disclosed herein, one or more mannanase variant or recombinant polypeptide or active fragment thereof described herein is used to hydrolyze galactomannans in a food (e.g., ice cream) manufacturing waste stream. Disclosed herein, a mannanase variant or recombinant polypeptide or active fragment thereof described herein is used for reducing the viscosity of residual food in processing equipment thereby facilitating cleaning after processing. Also disclosed herein, a mannanase variant or recombinant polypeptide or active fragment thereof described herein is used for reducing viscosity of print paste, thereby facilitating wash out of surplus print paste after textile printings. In general terms, a mannan-containing substrate is incubated with a mannanase variant or recombinant polypeptide or active fragment thereof described herein under conditions suitable for reducing the viscosity of the mannan-containing substrate.

In yet a further emboidment, one or more mannanase variant or recombinant polypeptide or active fragment thereof described herein can be used in the oil and gas industry to, for example, control the viscosity of drilling fluids; increase the rate at which the fluids used in hydraulic fracturing create subterranean fractures that exend from the borehole into the rock; clean the borehole filter cake; and combinations thereof.

### EXAMPLE 1

### Assays

The following assays are standard assays used in the examples described below. Occasionally specific protocols call for deviations from these standard assays. In those cases, deviations from these standard assay protocols below are identified in the examples.

### Performance Index

The performance index (PI) of an enzyme compares the performance of the variant (measured value) with the parent enzyme (theoretical value or measured value) at the same protein concentration. Theoretical values for the cleaning performance of the parent enzyme can be calculated using the parameters extracted from a Langmuir fit of a standard curve of the parent enzyme. A PI that is greater than 1 (PI>1) indicates improved performance by a variant as compared to the specified parent, such as, for example, PspMan4 (SEQ ID NO:2) or BspMan5 (SEQ ID NO: 16) while a PI of 1 (PI=1) identifies a variant that performs the same as the specified parent, and a PI that is less than 1 (PI<1) identifies a variant that performs worse than the specified parent.

### Protein Determination Assay

Protein concentration determination was performed using a high performance liquid chromatography (HPLC) method measuring integrated peak area to determine levels of protein expression in supernatants from cultures grown in 96-well micro-titer plates (MTPs). Samples were obtained from filtered culture supernatants and prepared as 4-fold dilutions in 25 mM Tris-HCl buffer, pH 7.5. Reversed-phase HPLC was carried out on an Agilent 1200 Series HPLC system equipped with a Poroshell 300SB-C8 column (2.1 × 75 mm) using a gradient elution composed of water and acetonitrile solvents, each supplemented with 0.1% TFA. Samples were eluted at 50°C, at a flow rate of 2 mL/min. Proteins were detected by measuring absorbance at 220 nm, and peaks were integrated using ChemStation software (Agilent Technologies). The protein concentration of samples was determined based on a standard curve of purified parent protein (e.g. PspMan4 (SEQ ID NO:2 or BspMan5 (SEQ ID NO:16)).

### Mannanase Activity Assay

The mannanase activity of the mannanase variants described in the Examples that follow were tested by measuring the hydrolysis of locust bean gum (LBG) galactomannan (Sigma G0753) in solution (approximately 0.3% (w/v) LBG substrate). The reagent solutions used were 50 mM Tris-HCl buffer, pH 7.5 (substrate dilution buffer), and 50 mM MOPS buffer, pH 7.2, containing 0.005% TWEEN^{®}-80 (enzyme dilution buffer). To prepare a working substrate solution, the LBG powder (Product No. G0753, Sigma-Aldrich, St. Louis, MO) was dissolved in a heated solution of 50 mM Tris-HCl buffer, pH 7.5, under stirring. Upon cooling to room temperature, the solution was centrifuged and the clear supernatant was used as the substrate solution.

Enzymes were diluted into enzyme dilution buffer (50 mM MOPS, pH 7.2, 0.005% TWEEN^{®}-80) and aliquots of the diluted enzyme solutions were added to the wells of a flat-bottom clear polystyrene MTP containing the LBG substrate solution. The plates were sealed and incubated at 40°C with agitation at 900 rpm for 10 min (e.g. in an iEMS incubator/shaker, Thermo Fisher Scientific, Waltham, MA).

After incubation, the released reducing sugars were quantified using the BCA reagent assay (Catalog No. 23225, Thermo Scientific Pierce, Rockford, IL). Specifically, aliquots from each well of the LBG assay plate were added to a PCR plate containing BCA working reagent solution (prepared according to the manufacturer's instructions); the sample to working reagent ratio was 1:9 (v/v). The plates were sealed and incubated in a thermocycler (e.g. Tetrad2 Peltier Thermal Cycler, Bio-Rad Laboratories, Hercules, CA) at 95°C for 2-3 min.

After the plate cooled to 30°C, the reaction solution was transferred to a fresh flat-bottom clear polystyrene MTP (e.g. Costar 9017) and absorbance was measured at 562 nm in a plate reader spectrophotometer (e.g. SpectraMax Plus 384, Molecular Devices, Sunnyvale, CA). The absorbance value of a sample not containing mannanase (blank) was subtracted from the absorbance values of the mannanase-containing samples. The resulting absorbance was taken as a measure of mannanase activity. The specific activity of the parent mannanase and variants thereof was calculated by dividing the resulting absorbance by the protein concentration calculated from the protein determination assay. Mannanase activity PI values were calculated by dividing the mannanase specific activity of the variants by that of the parent.

### Stability Assay

The stability of the mannanase variants described in the Examples that follow was tested under the stress condition in 50 mM MOPS buffer, pH 7.2, 0.005% TWEEN^{®}-80 at 57°C by measuring the residual activity of samples after incubation at elevated temperature for 5 min. To measure the initial (unstressed) activity, the enzyme samples were diluted in 50 mM MOPS buffer, pH 7.2, 0.005% TWEEN^{®}-80 and assayed immediately for activity on LBG using the assay described under "Mannanase Activity Assay" section above. To measure the stressed activity, the diluted enzyme samples in 50 mM MOPS buffer, pH 7.2, 0.005% TWEEN^{®}-80 were incubated in a sealed PCR plate at 57°C for 5 min in a thermocycler (Tetrad2 Peltier Thermal Cycler, Bio-Rad Laboratories, Hercules, CA), then assayed for activity as described in the "Mannanase Activity Assay" section above.

### Residual Activity Calculation

Once stressed and unstressed activity values were measured by hydrolysis of LBG substrate as described above, the % residual activities were calculated by taking a ratio of the stressed to unstressed activity and multiplying by 100. Stability PI values were obtained by dividing the residual activity of variants by that of the parent.

### Cleaning performance of mannanase variants using Microswatch Assay

Variants were tested for cleaning performance on LBG microswatches (CFT C-S-73, Center for Testmaterials, Vlaardingen, The Netherlands) relative to performance of parent.

Cleaning performance was measured using a high throughput assay developed to measure galactomannan removal from technical soils. The assay measures the release of LBG from the technical soils containing LBG. The BCA reaction using a commercially available reagent (Catalog No. 23225, Thermo Scientific Pierce, Rockford, IL) is used to measure reducing ends of oligosaccharides in solution in the presence of enzyme, compared to a blank control. This measurement correlates with the cleaning performance of the enzyme. As the mannanase hydrolyzes galactomannans, oligosaccharides of varying lengths with reducing ends are presumably released from the cotton swatch. The bicinchoninic acid in the BCA reagent then allows for the highly sensitive colorimetric detection of Cu¹⁺ formed by the reduction of Cu²⁺.

Two 5.5 cm diameter LBG microswatches (CFT C-S-73, Center for Testmaterials, Vlaardingen, The Netherlands) were placed into each well of a flat-bottom, non-binding 96-well assay plate (e.g. Corning 3641). Enzymes were diluted into 50 mM MOPS buffer, pH 7.2, containing 0.005% TWEEN^{®}-80. Microswatch assay buffer (25 mM HEPES, pH 8, 2 mM CaCl₂, 0.005% TWEEN^{®}-80) and aliquots of diluted enzymes were added into each well of the 96-well microswatch assay plate for a combined volume of 100 µL. Plates were sealed and incubated at 25°C with agitation at 1150 rpm for 20 min (e.g. in an iEMS incubator/shaker, Thermo Fisher Scientific, Waltham, MA).

After the incubation, the released reducing sugars were quantified using the BCA reagent assay as described in the "Mannanase Activity Assay" section above. The resulting absorbance was taken as a measure of cleaning performance. Cleaning performance PI values were calculated by dividing the cleaning performance of variants by that of the parent at the same protein concentration. As stated in the "Performance Index" section above, theoretical values for the cleaning performance of the parent at the relevant protein concentrations were calculated using the parameters extracted from a Langmuir fit of measured values for a standard curve of the parent.

### EXAMPLE 2

### Generation of a Site Evaluation Library of PspMan4

Site evaluation libraries (SELs) for PspMan4 were generated using standard molecular biology protocols to introduce single amino acid substitutions into the PspMan4 protein sequence. A template plasmid containing the *PspMan4* gene (SEQ ID NO: 1) was constructed. SELs were produced at preselected positions in the mature region of PspMan4 (SEQ ID NO:2). Forward and reverse NNS oligomers for each amino acid site in the SELs and the outside primers (hybridizing to the start or end of the expression cassette) were ordered from Eurofins Genomics, Huntsville, AL, USA.

The expression cassette consisted of the promoter (SEQ ID NO:3) and signal peptide (SEQ ID NO:4) from the *B. subtilis aprE* gene, the *PspMan4* gene (SEQ ID NO: 1), and the terminator (SEQ ID NO: 5) from the *B. amyloliquefaciens* BPN' gene. Polymerase chain reactions (PCRs) with appropriate primer pairs and the template plasmid were performed to generate the variant genes.

The PCR fragments were assembled and a suitable *B. subtilis* strain was transformed with the assembled DNA. The transformed cells were plated on Luria's Agar with 5 ppm chloramphenicol. For each library, single bacterial colonies were picked and grown in Luria's broth with 5 ppm chloramphenicol selection for subsequent DNA isolation and gene sequence analysis. The nucleotide sequence for each of the variants was confirmed by Next Generation DNA analysis (Illumina, San Diego, CA).

To generate samples of PspMan4 parent and variants thereof for biochemical characterization, selective growth of the mannanases was performed in 96-well MTPs at 37°C for 68 hours in cultivation medium (enriched semi-defined media based on MOPs buffer, with urea as major nitrogen source, glucose as the main carbon source, and supplemented with 1% soytone for robust cell growth) contained in each well. Cultures were harvested by centrifugation at 3600 rpm for 45 min and filtered through Multiscreen^{®} filter plates (EMD Millipore, Billerica, MA, USA) using a Millipore vacuum system. The filtered culture supernatants were used for the assays described above in Example 1.

The nucleic acid sequence for the *PspMan4* gene used to generate the SELs is set forth as SEQ ID NO: 1. The amino acid sequence of the PspMan4 protein encoded by the *PspMan4* gene is set forth as SEQ ID NO:2. The nucleic acid sequence for the *aprE* promoter from *B. subtilis* is set forth as SEQ ID NO:3. The nucleic acid sequence for the *aprE* signal peptide from *B. subtilis* is set forth as SEQ ID NO:4. The nucleic acid sequence for aprE Terminator from *B. subtilis* is set forth as SEQ ID NO:5.

### EXAMPLE 3

### Evaluation of PspMan4 Mutations

Combinable mutations can be described as those substitutions in a molecule that can be used to make combinatorial variants. Combinable mutations are ones that improve at least one desired property of the molecule, while not significantly decreasing either: expression, activity, or stability. Productive positions are described as those positions within a molecule that are most useful for making combinatorial variants exhibiting an improved characteristic, where the position itself allows for at least one combinable mutation.

Clarified culture supernatant samples for PspMan4 variants were tested using the methods described in Example 1: Mannanase Activity Assay, Stability Assay, Cleaning Performance Assay, and Protein Concentration. PI values were calculated as described in Example 1, using PspMan4 as the parent (SEQ ID NO:2) for comparison.

Combinable mutations in PspMan4 were identified using the following criteria: a) protein expression >140 ppm; b) PI ≥ 0.7 for all of mannanase activity, cleaning performance, and stability; and c) PI for at least one of mannanase activity, cleaning performance, and stability ≥1.0. The sites in PspMan4 that meet the combinable mutation criteria are set forth in Table 1. Productive positions in PspMan4 include: 10, 19, 38, 59, 60, 62, 63, 66, 67, 68, 70, 71, 74, 75, 78, 79, 80, 97, 129, 131, 135, 136, 143, 167, 168, 184, 213, 214, 225, 228, 235, 242, 244, 258, 259, 261, and 283, wherein the amino acid positions of PspMan4 are numbered by correspondence with the amino acid sequence of SEQ ID NO:2.

| **Table 1: Productive Positions and PI for Combinable Mutations in PspMan4** | | | | |
|---|---|---|---|---|
| **Amino Acid Position** | **Substitution** | **Performance Index (PI)** | | |
| | | **Cleaning Performance Assay** | **Stability Assay** | **Mannanase Activity Assay** |
| 10 | N10T | 1.0 | 0.8 | 0.8 |
| 10 | N10Q | 1.1 | 0.9 | 1.0 |
| 19 | P19V | 1.0 | 0.9 | 1.0 |
| 19 | P19E | 1.1 | 2.4 | 1.0 |
| 38 | T38I | 1.2 | 2.4 | 0.8 |
| 38 | T38Q | 1.2 | 0.8 | 1.1 |
| 38 | T38R | 0.8 | 0.9 | 1.1 |
| 38 | T38V | 1.2 | 2.5 | 0.8 |
| 38 | T38E | 1.2 | 2.1 | 0.8 |
| 38 | T38M | 1.1 | 0.9 | 1.0 |
| 38 | T38L | 1.0 | 1.8 | 0.8 |
| 59 | S59N | 1.0 | 0.8 | 1.2 |
| 59 | S59G | 1.0 | 0.8 | 1.2 |
| 59 | S59D | 1.1 | 3.0 | 1.1 |
| 59 | S59K | 1.1 | 0.7 | 1.1 |
| 59 | S59T | 1.2 | 1.6 | 1.3 |
| 59 | S59Q | 1.1 | 2.2 | 1.1 |
| 60 | L60F | 0.7 | 1.3 | 0.9 |
| 60 | L60M | 1.0 | 1.1 | 1.0 |
| 60 | L60V | 1.0 | 2.5 | 1.1 |
| 62 | T62V | 1.4 | 2.6 | 1.0 |
| 62 | T62I | 1.3 | 1.4 | 1.0 |
| 62 | T62Q | 1.3 | 1.7 | 1.0 |
| 62 | T62E | 1.2 | 4.1 | 1.0 |
| 63 | K63L | 1.4 | 2.5 | 0.8 |
| 66 | L66T | 1.2 | 1.1 | 1.1 |
| 66 | L66V | 0.9 | 1.0 | 1.5 |
| 66 | L66C | 1.1 | 2.2 | 1.0 |
| 67 | N67Q | 1.2 | 0.9 | 1.0 |
| 67 | N67P | 0.9 | 1.5 | 1.2 |
| 67 | N67G | 1.1 | 0.7 | 0.8 |
| 67 | N67A | 1.0 | 0.8 | 1.1 |
| 67 | N67V | 1.0 | 0.8 | 1.0 |
| 67 | N67D | 1.0 | 3.4 | 0.8 |
| 67 | N67E | 0.8 | 3.0 | 0.7 |
| 67 | N67S | 1.0 | 1.1 | 1.2 |
| 68 | A68W | 1.3 | 0.8 | 1.0 |
| 68 | A68R | 1.1 | 2.0 | 0.9 |
| 68 | A68L | 1.4 | 1.1 | 0.8 |
| 68 | A68M | 1.2 | 1.6 | 0.8 |
| 68 | A68S | 1.1 | 2.3 | 0.8 |
| 70 | K70V | 1.4 | 1.0 | 1.2 |
| 70 | K70R | 1.3 | 0.9 | 1.0 |
| 71 | N71H | 1.3 | 1.5 | 1.2 |
| 71 | N71D | 1.2 | 1.5 | 1.1 |
| 74 | N74Q | 1.2 | 1.0 | 1.1 |
| 74 | N74V | 1.1 | 1.0 | 1.3 |
| 74 | N74C | 1.2 | 2.0 | 1.1 |
| 74 | N74E | 1.2 | 3.3 | 1.0 |
| 75 | V75I | 0.9 | 1.5 | 0.8 |
| 78 | Q78L | 1.1 | 0.7 | 1.0 |
| 78 | Q78D | 1.1 | 2.7 | 0.7 |
| 78 | Q78M | 1.2 | 0.8 | 0.9 |
| 78 | Q78A | 1.1 | 1.1 | 1.0 |
| 79 | N79E | 1.2 | 1.0 | 0.8 |
| 79 | N79W | 1.3 | 0.7 | 1.0 |
| 79 | N79F | 1.4 | 0.7 | 1.0 |
| 80 | K80Q | 1.2 | 1.1 | 1.3 |
| 80 | K80T | 1.4 | 9.1 | 1.1 |
| 97 | N97E | 1.3 | 2.4 | 1.2 |
| 97 | N97Q | 1.2 | 0.8 | 1.1 |
| 97 | N97L | 1.7 | 0.9 | 1.5 |
| 97 | N97P | 1.3 | 1.2 | 1.4 |
| 129 | Y129M | 1.1 | 2.1 | 1.4 |
| 131 | T131P | 0.9 | 1.1 | 1.2 |
| 135 | S135A | 1.1 | 1.3 | 1.0 |
| 135 | S135Q | 1.1 | 1.2 | 0.9 |
| 135 | S135C | 1.0 | 1.0 | 0.8 |
| 136 | A136E | 1.2 | 1.4 | 0.9 |
| 143 | K143Q | 1.2 | 1.0 | 0.9 |
| 143 | K143R | 1.1 | 0.9 | 1.0 |
| 167 | F167S | 0.9 | 1.8 | 1.0 |
| 167 | F167Y | 1.1 | 2.1 | 0.8 |
| 167 | F167W | 0.9 | 2.4 | 0.7 |
| 167 | F167L | 1.1 | 1.5 | 0.7 |
| 168 | P168E | 1.0 | 0.8 | 1.1 |
| 168 | P168L | 1.1 | 1.1 | 1.0 |
| 168 | P168M | 1.0 | 1.0 | 1.2 |
| 168 | P168G | 1.3 | 2.5 | 1.6 |
| 168 | P168S | 1.3 | 3.3 | 1.2 |
| 168 | P168T | 1.3 | 3.9 | 1.2 |
| 168 | P168A | 1.2 | 5.5 | 1.3 |
| 184 | Q184L | 1.2 | 2.4 | 1.0 |
| 184 | Q184M | 1.2 | 1.3 | 0.9 |
| 184 | Q184F | 1.0 | 0.7 | 1.0 |
| 184 | Q184H | 1.0 | 0.7 | 0.9 |
| 184 | Q184D | 1.0 | 1.6 | 0.9 |
| 184 | Q184P | 0.9 | 1.8 | 0.9 |
| 213 | N213E | 1.3 | 0.7 | 1.2 |
| 214 | K214C | 1.5 | 0.7 | 1.4 |
| 214 | K214Q | 1.3 | 1.3 | 1.2 |
| 225 | G225P | 0.7 | 3.1 | 0.7 |
| 225 | G225W | 1.0 | 1.2 | 0.7 |
| 225 | G225C | 1.0 | 2.9 | 1.0 |
| 225 | G225A | 0.9 | 1.1 | 0.9 |
| 228 | T228G | 0.9 | 1.4 | 0.8 |
| 228 | T228K | 0.7 | 1.5 | 0.9 |
| 228 | T228A | 1.0 | 1.1 | 0.9 |
| 228 | T228V | 1.1 | 1.1 | 1.2 |
| 228 | T228S | 1.2 | 0.8 | 1.0 |
| 228 | T228I | 1.2 | 1.0 | 1.3 |
| 228 | T228Y | 1.1 | 0.8 | 1.2 |
| 228 | T228H | 0.9 | 1.1 | 1.1 |
| 235 | Y235S | 1.0 | 1.0 | 1.1 |
| 235 | Y235G | 1.1 | 1.0 | 1.1 |
| 235 | Y235V | 1.2 | 0.8 | 1.2 |
| 235 | Y235Q | 1.1 | 0.7 | 0.9 |
| 235 | Y235I | 1.2 | 0.7 | 0.9 |
| 235 | Y235L | 1.2 | 1.4 | 1.1 |
| 242 | Q242S | 1.2 | 0.8 | 1.3 |
| 242 | Q242E | 1.2 | 0.7 | 1.3 |
| 244 | K244S | 1.3 | 1.5 | 1.6 |
| 244 | K244A | 1.4 | 0.9 | 1.5 |
| 244 | K244G | 1.1 | 1.4 | 1.5 |
| 244 | K244L | 1.2 | 4.8 | 1.5 |
| 244 | K244C | 1.3 | 1.3 | 1.0 |
| 244 | K244M | 1.3 | 3.8 | 1.6 |
| 244 | K244P | 1.4 | 5.6 | 1.3 |
| 258 | S258T | 1.2 | 0.8 | 1.2 |
| 258 | S258G | 1.2 | 1.0 | 1.1 |
| 258 | S258N | 1.2 | 1.2 | 1.0 |
| 258 | S258A | 1.1 | 1.6 | 1.0 |
| 258 | S258E | 1.3 | 1.6 | 1.1 |
| 258 | S258M | 1.3 | 0.9 | 1.1 |
| 258 | S258D | 1.3 | 1.9 | 0.9 |
| 258 | S258P | 1.2 | 1.8 | 0.9 |
| 259 | G259A | 1.5 | 0.8 | 1.3 |
| 259 | G259W | 1.5 | 0.7 | 1.6 |
| 259 | G259R | 1.1 | 0.9 | 1.2 |
| 259 | G259E | 1.5 | 1.6 | 1.1 |
| 259 | G259S | 1.3 | 0.7 | 1.1 |
| 261 | N261M | 1.6 | 1.0 | 1.7 |
| 261 | N261W | 1.6 | 0.9 | 1.7 |
| 261 | N261P | 1.0 | 1.6 | 0.8 |
| 261 | N261T | 1.3 | 1.0 | 1.0 |
| 261 | N261V | 1.9 | 1.0 | 1.6 |
| 261 | N261I | 1.3 | 0.8 | 1.2 |
| 261 | N261Y | 1.4 | 0.8 | 1.6 |
| 261 | N261Q | 1.1 | 1.5 | 1.1 |
| 261 | N261R | 1.1 | 2.7 | 0.9 |
| 261 | N261S | 1.2 | 1.6 | 1.1 |
| 283 | D283H | 1.4 | 0.8 | 1.2 |
| 283 | D283T | 1.5 | 2.0 | 1.3 |
| 283 | D283G | 1.5 | 2.6 | 1.3 |

As shown in Table 1, multiple substitutions that were beneficial in one or more properties were observed at nearly all these sites. In numerous cases, when multiple combinable substitutions (such as those listed on Table 1) were introduced into the PspMan4 sequence, the resulting mannanases showed improved stability and cleaning performance when compared to the parent molecule. Combinatorial variants comprising two or more of these sites are further described in patent application numbers 62/251,516, filed November 5, 2015 and 62/278,387, filed January 13, 2016.

### EXAMPLE 4

### Crystallographic Structures of PspMan4 Variants

The three-dimensional structures of two PspMan4 variants, PspMan118 and PspMan148, were determined using X-ray crystallographic method.

PspMan118 (SEQ ID NO:6) with mutations: P19E/T38E/N67D/N97D/Y129M/ P168S/Q184L/K244L/S258D/N261R (wherein the amino acid positions are numbered by correspondence with the amino acid sequence of SEQ ID NO:2), was crystallized using the hanging drop method starting with a 1% protein solution in 50 mM MES buffer, pH 6.0 with 50 mM sodium chloride. The reservoir solution contained 0.7M sodium phosphate, 0.8M potassium phosphate and 0.1M HEPES pH 7.5. Crystals grew in the space group P2₁2₁2₁ having one molecule in the asymmetric unit with unit cell dimensions a=53.2 Å, b=76.7 Å, and c=77.3Å.

PspMan148 (SEQ ID NO:7) with mutations: N10T/P19E/S30T/T38E/S59V/L60Q/ K63R/N67D/N97D/Y129M/K143Q/P168S/Q184L/G225P/T228V/Y23SL/K244L/S258D/ N261R/Z298.01Q (wherein the amino acid positions are numbered by correspondence with the amino acid sequence of SEQ ID NO:2), was crystallized using the hanging drop method starting with a 1% protein solution in 50 mM MES buffer, pH 6.0 with 50 mM sodium chloride. The reservoir solution contained 16% 2-propanol 0.16M calcium chloride, and 80 mM sodium acetate, pH 4.6. Crystals grew in the space group P2₁2₁2₁ having one molecule in the asymmetric unit with unit cell dimensions a=52.8 Å, b=77.0 Å, and c=78.5Å.

Data for PspMan118 and PspMan148 crystals were collected on a Bruker X8 Proteum diffraction system to a resolution of 1.8 Å and 1.7 Å, respectively. Additional statistics for data collection are presented in Table 2.

The structure of PspMan118 was determined using molecular replacement with the coordinates of residues 27-326 from *Bacillus sp.* JAMB-602 mannanase (accession number BAD99527.1, PDB entry 1WKY_A) as a starting model. The model was fitted using the Coot software package [Emsley, P. et al (2010), Acta Cryst. D; 66:486-501].

The structure of PspMan148 was determined using molecular replacement with the coordinates of PspMan118 as a starting model. The coordinates were adjusted to accommodate the electron density for the additional substitutions and fitted using the Coot software package. Sparse, weak density was observed for the additional residue, Q, inserted at the C-terminus of PspMan148. After fitting and refitting adjustments, the coordinates for both structures were refined using the REFMAC program with standard default settings in the CCP4 software suite.

The final models had good stereochemistry as reported in Table 3. For reference, the coordinates of the PspMan118 and PspMan148 variants could be aligned with an overall rms (root mean square) deviation of 0.133Å for 1954 common atoms.

| **Table 2: Data collection statistics for PspMan118 and PspMan148 Variants** | | |
|---|---|---|
| | **PspMan118** | **PspMan148** |
| Wavelength | 1.54Å | 1.54Å |
| Space group | P2₁2₁2₁ | P2₁2₁2₁ |
| Molecule/asymmetric unit | 1 | 1 |
| Unit cell dimensions | 53.2, 76.7, 77.3 Å | 52.8, 77.0, 78.5 Å |
| Resolution | 1.8Å | 1.7Å |
| Unique reflections | 27826 | 32080 |
| Multiplicity | 5.8 (1.8) | 2.5 (1.4) |
| Completeness | 98.8% | 97.9% |
| R merge | 0.04 (0.14)* | 0.05 (0.10) |
| I/σ | 25.4 (4.7) | 14.4 (4.8) |

| | | |
|---|---|---|
| *Values for the outer shell are presented in parenthesis | | |

| **Table 3: Statistics of the Refined Model for PspMan118 and PspMan148 Variants** | | |
|---|---|---|
| | **PspMan118** | **PspMan148** |
| R work | 0.16 | 0.15 |
| R free | 0.20 | 0.18 |
| No. protein residues | 297 | 298 |
| No. atoms | 2277 | 2288 |
| rmsd bond lengths | 0.0196Å | 0.0138Å |
| rmsd bond angles | 1.86° | 1.55° |

The coordinates of the PspMan118 monomers superpose with the catalytic domains of two other mannanase structures: *Bacillus sp.* strain JAMB-602 mannanase (PDB entry 1WKY_A) and *B. agaradhaerens* strain NCIMB 40482 mannanase (PDB entry 2WHL_A), with an overall rms deviation of 0.38Å and 0.42Å, respectively, using all common atoms. Thus, even though these three enzymes only share about 60% amino acid sequence identity over residues 1 to 295 of PspMan4, all three mannanases share a common fold for the catalytic domains.

Figure 1 depicts a structural comparison of the 1WKY_A mannanase to the PspMan118 mannanase variant, where the main chain folding of 1WKY_A (shown in grey) is compared to the main chain folding of PspMan118 (shown in black). Figure 1 shows that PspMan118 shares a common cation binding site with 1 WKY A, and that 1WKY_A has an additional carbohydrate binding domain. The cation binding site that PspMan118 shares with 1WKY_A is formed by the carbonyl oxygen of Gly225 residue, the side chain of Asp231, the carbonyl oxygen of Thr232, and the side chain of Glu234.

PspMan118 and PspMan148 can be further characterized by two motifs: (i) an NDL motif at positions N34D35L36, and (ii) a deletion motif spanning positions 263-274 (wherein the amino acid positions are numbered by correspondence with the amino acid sequence of SEQ ID NO:2) relative to other GH5 mannanase sequences such as those exemplified by 1WKY_A) and 2WHL_A. Figure 2 depicts a further structural comparison of PspMan118 to 1WKY_A, wherein this comparison shows that the residues encompassing the NDL and Deletion motifs of PspMan1 18 are in close proximity to each other.

The *B. agaradhaerens* 2WHL _A mannanase structure has been reported as a mannotriosyl-enzyme complex. The structure of PspMan148 was aligned with 2WHL_A to study the location of the variant sites with respect to the mannotriose bound in the active site. PspMan148 was chosen for comparison as it includes all 10 substitutions present in PspMan118, as well as nine additional substitutions and one insertion at the C-terminus. As with PspMan1 18, it is possible to align the structure of PspMan148 with that of 2WHL_A, resulting in an overall rms deviation of 0.405Å for 1660 common atoms. The superposition of the PspMan148 and 2WHI, structures is depicted in Figures 3A-3C.

In Figure 3A, the main chain folding of 2WHL_A is schematically represented in light gray and mannotriose is shown as light gray sticks. The main chain of PspMan148 is shown in black with the side chains of the nineteen substituted amino acids shown as black stick figures. The amino acidZ298.01Q inserted in PspMan148, which was disordered in the electron density map, is not included in this figure.

Seven of the nineteen substitutions in PspMan148 are situated in the substrate binding site. These include S30T, S59V, L60Q, K63R, T228V, S258D and N261R (wherein the amino acid positions are numbered by correspondence with the amino acid sequence of SEQ ID NO:2). Figure 3B shows the superposition of the PspMan148 and 2WHL_A structures with the substrate binding site substitutions shown as black spheres. Among these substitutions, L60Q introduces a side chain that can be seen at homologous positions in both the 1WKY_A and 2WHL_A structures.

In Figure 3B, the mannotriosyl moiety bound to the mannanase in the 2WHL_A structure is shown as gray sticks to indicate the relative location of the substrate binding site. The positions of the seven substitutions (S30T, S59V, L60Q, K63R, T228V, S258D and N261R) around and near the substrate binding site in PspMan148 are shown as black spheres.

As seen in Figure 3C, the remaining twelve substitutions in PspMan148 are distributed on the surface of the molecule (shown as black spheres). Of these twelve substitutions, Q184L and G225P are of particular interest. The Q184L substitution introduces a leucine side chain that shields a salt bridge between Arg149 and Glut 82, thereby stabilizing the protein. The G225P substitution introduces a rigidifying proline residue where the main chain carbonyl oxygen forms a ligand to the cation (a calcium ion in PspMan148), thereby potentially stabilizing the bound calcium, which would make the enzyme less sensitive to chelants present in detergent formulations.

Figures 4A-B depict the multiple sequence alignment using MUSCLE software of the mannanase domains of PspMan4 (SEQ ID NO:2), PspMan148 (SEQ ID NO:7), BspMan5 (SEQ ID NO:16), US6566114-002 (residues 32-330)(SEQ ID NO:15), US6566114-002 (residues 32-340)(SEQ ID NO:17), WO2015022428-0015 (SEQ ID NO:8), and 2WHL_A (SEQ ID NO:9) with productive positions in PspMan4 being underlined and in bold font. Considering the strong structural similarities among these mannanases, it might be expected that introducing the substitutions that confer improvement in PspMan4 at structurally homologous sites in other homologous GH5 mannanases such as the 1WKY_A or 2WHL_A mannanases could confer similar improvements in performance and/or stability to these molecules.

To evaluate the effect of substitutions identified as productive in the PspMan4 backbone in other backbones, the same substitutions: P19E, T38E, H67D, F129M, P168S, Q184L, H225P, R244L, P258D, E261R were introduced in the BspMan5 mannanase (SEQ ID NO:16) backbone as described below in Example 5.

### EXAMPLE 5

### Cloning and Heterologous Expression of BspMan5 and Variants Thereof

BspMan5 (SEQ ID NO:16) is a variant of US6566114-002 (residues 32-340)(SEQ ID NO:17), wherein the amino acid sequence of BspMan5 has mutation: P85L and amino acids AGK inserted at the N-terminus, wherein the amino acid positions are numbered by correspondence with the amino acid sequence of SEQ ID NO:2.

The gene encoding the BspMan5 (SEQ ID NO:16) mannanase was synthesized and inserted into expression vector p2JM103BBI (Vogtentanz, Protein Expr Purif, 55:40-52, 2007) by Generay (Shanghai, China), resulting in an expression vector containing: an aprE promoter, an aprE signal sequence (used to direct target protein secretion in *Bacillus subtilis*) an oligonucleotide named AGK-proAprE that encodes peptide Ala-Gly-Lys to facilitate the secretion of the target protein, and the synthetic nucleotide sequence encoding the mature region of the mannanase gene of interest. Generay (Shanghai, China) produced ten single point variants of the BspMan 5 parental gene, and cloned them into the same expression vector.

A suitable *B. subtilis* host strain was transformed with each of the expression plasmids and the transformed cells were spread on Luria Agar plates supplemented with 5ppm chloramphenicol. To produce each of the mannanases, *B. subtilis* transformants containing the plasmids were grown in a 250 ml shake flask in a MOPS based defined medium, supplemented with additional 5 mM CaCl₂.

The nucleotide sequence of the parental BspMan5 mannanase gene inserted in the expression plasmid is set forth as SEQ ID NO: 18. The gene has an alternative start codon (GTG) and an oligonucleotide encoding the three residue addition (AGK) at the 5' end of the gene. The amino acid sequence of the BspMan5 precursor protein expressed from the p2JM plasmid is set forth as SEQ ID NO: 19. The amino acid sequence of the predicted mature protein, BspMan5, expressed from the plasmid is set forth as SEQ ID NO: 16.

BspMan5 variants were generated by making point mutations on the BspMan5 gene using molecular biology techniques known in the art. The properties of each of the variants were explored in subsequent examples. The list of BspMan5 variants is set forth in Table 4 with the substitutions listed relative to each specifed parent, wherein the amino acid positions are numbered by correspondence with the amino acid sequence of SEQ ID NO:2. The amino acid sequences of the mature BspMan5 variants: BspMan 6-15 are set forth in SEQ ID NOs: 20-29.

| **Table4: BspMan5 Variants With Sequence Substitutions Relative to Parent** | | | |
|---|---|---|---|
| **SEQ ID NO** | **Mannanase** | **Sequence Substitutions Relative to BspMan5** | **Sequence Substitutions Relative to residues 32-340 of** US6566114-002 |
| 16 | BspMan5 | None | P85L |
| 20 | BspMan6 | P19E | P19E-P85L |
| 21 | BspMan7 | T38E | T38E-P85L |
| 22 | BspMan8 | H67D | H67D-P85L |
| 23 | BspMan9 | F129M | P85L-F129M |
| 24 | BspMan10 | P168S | P85L-P168S |
| 25 | BspMan11 | Q184L | P85L-Q184L |
| 26 | BspMan12 | H225P | P85L-H225P |
| 27 | BspMan13 | R244L | P85L-R244L |
| 28 | BspMan14 | P258D | P85L-P258D |
| 29 | BspMan15 | E261R | P85L-E261R |

### Purification of BspMan5 and variants thereof

BspMan 5-15 were purified from clarified *B. subtilis* culture broth. A combination column chromatography including ion exchange, hydrophobic interaction or sizing fractionation resins was used. The fractions containing the mannanase were identified by testing their activities on LBG via the PAHBAH (p-hydroxy benzoic acid hydrazide) assay (Lever, Anal Biochem, 47:248, 1972). The fractions of interest were pooled, concentrated using a 10K Amicon Ultra device, and the samples were adjusted to 40% glycerol and stored at -20°C for long-term storage.

### EXAMPLE 6

### Thermostability of BspMan5 and Variants Thereof

Thermostability was evaluated by determining the T50 value, which is defined as the temperature at which the enzyme retains 50% activity under the conditions of the assay. Each enzyme was incubated for 2 hours in a thermocycler in 50 mM sodium citrate buffer pH 6.0 containing 0.005% Tween-80, at the following temperatures: 40, 41.7, 44.7, 49.4, 55, 59.7, 63, 65, 67, and 70°C. The activity of the enzymes was measured using LBG as the substrate (0.45% LBG solution in 50 mM sodium citrate buffer pH 6.0) after a 10 min incubation at 50°C. The released reducing sugar was quantified in a PAHBAH (p-Hydroxy benzoic acid hydrazide) assay (Lever, Anal. Biochem, 47:273, 1972). Aliquots of each enzyme sample that were maintained on ice were tested to determine the 100% activity values. The T50 temperature values were measured after a 2 hour incubation to determine the remaining activity. The data was plotted to determine the T50 value for each enzyme sample, shown in Table 5.

| **Table 5: Thermostability of BspMan5 and Variants Thereof** | |
|---|---|
| Mannanase | T50 °C |
| BspMan5 | 62.5 |
| BspMan6 | 61 |
| BspMan7 | 62 |
| BspMan8 | 64 |
| BspMan9 | 64 |
| BspMan10 | 64 |
| BspMan11 | 63 |
| BspMan12 | 64 |
| BspMan13 | 61 |
| BspMan14 | 61 |
| BspMan15 | 61 |

### EXAMPLE 7

### The Cleaning Performance of BspMan5 and Variants Thereof

The cleaning performance of BspMan 5-15 mannanases was assessed in a microswatch assay which measures the release of LBG from a technical soil. The released reducing sugar was quantified using the PAHBAH (p-Hydroxy benzoic acid hydrazide) assay (Lever, Anal. Biochem, 47:273, 1972). Two CS-73 microswatches (5.5 cm in diameter) (CFT, Vlaardingen, Holland) were placed into each well of a flat-bottom, non-binding 96-well assay plate. Enzyme samples were diluted in deionized water. Table 6 lists the detergents used, and the place of purchase. Table 7 lists the detergent conditions used, including: dose, pH, buffer system (if used), hardness (3:1 Ca:Mg; ppm), temperature (°C) and wash time (min) for the cleaning assays. Diluted enzyme at a final dose of 2.5 ppm and detergent solution was added into each well to a total volume of 100 µl. Plates were sealed and incubated under respective cleaning conditions of the detergents. After the cleaning evaluation time ended, 10 µl of each reaction mixture was transferred to a PCR plate containing 100 µl PAHBAH solution per well. Plates were sealed and incubated in a PCR machine at 95°C for 5 min. Later, the plate was cooled to 25°C, 80 µl of the supernatant was transferred to a fresh flat-bottom MTP, and the absorbance at 405 nm was measured in a spectrophotometer. The increase in absorbance at 405 nm is used as a measure of cleaning performance on the mannan-based CS-73 microswatches. Results are shown on Table 8 as PI values, which were calculated by taking the ratio of the absorbance of the variant to the value observed for the BspMan5 parent enzyme.

| **Table 6: Commercial Laundry Detergents** | | | | |
|---|---|---|---|---|
| **Detergent name** | **Type** | **Manufacturer** | **Country of Purchase** | **Year Purchased** |
| Kirkland Signature Ultra Clean | HDL | Private label | USA | 2016 |
| Una Color | HDL | Private label | Netherlands | 2016 |
| ECE-2 without bleach | HDD | WFK | Germany | 2015 |

| **Table 7: Conditions for Cleaning Assays** | | | | | |
|---|---|---|---|---|---|
| **Detergent name** | **Detergent dose** | **pH; buffersystem** | **Hardness (3:1 Ca:Mg, ppm)** | **T (°C)** | **Wash time (min)** |
| Kirkland Signature Ultra Clean | 0.71 g/L | 8.23; 5 mM HEPES | 150 | 20 | 15 |
| Una color | 7.5 mL/L | 8.19; 5 mM HEPES | 250 | 25 | 15 |
| ECE-2 without bleach | 2 g/L | 10.97 | 150 | 20 | 20 |

| **Table 8: Stain Removal Results for CS-73 LBG shown as PI compared to BspMan5 Parent** | | | |
|---|---|---|---|
| **Mannanase** | **Una Color** | **Kirkland Signature Ultra Clean** | **ECE2 without bleach** |
| BspMan5 | 1.0 | 1.0 | 1.0 |
| BspMan6 | 1.0 | 1.1 | 1.1 |
| BspMan7 | 1.1 | 1.0 | 1.0 |
| BspMan8 | 1.1 | 1.1 | 1.0 |
| BspMan9 | 0.9 | 1.0 | 1.0 |
| BspMan10 | 0.9 | 0.9 | 1.0 |
| BspMan11 | 1.0 | 1.0 | 1.1 |
| BspMan12 | 1.0 | 1.2 | 1.1 |
| BspMan13 | 1.0 | 1.2 | 1.1 |
| BspMan14 | 1.0 | 1.1 | 1.0 |
| BspMan15 | 1.0 | 1.1 | 1.0 |

## Claims

1. A mannanase variant comprising an amino acid sequence comprising one or more variations versus SEQ ID NO:2 selected from:
X19E, X38E, X67D, X129M, X168S, X184L, X225P, X244L, X258D, and X261R;
wherein X is any amino acid;
wherein the amino acid positions of said variant are numbered by correspondence with the amino acid sequence of SEQ ID NO:2;
wherein the mannanase variant has at least 90% identity to SEQ ID NO: 2, and
wherein the mannanase variant has one or more improved property over a reference polypeptide having the amino acid sequence of SEQ ID NO: 2, wherein the improved property is selected from thermal stability, detergent stability, specific activity towards a mannan substrate, and cleaning performance as determined using the Microswatch Assay described in Example 1,
with the proviso that the mannanase variant does not have the amino acid sequence set forth as any one of SEQ ID NOs: 62, 63, 65, 66, 67, 80 and 82 in WO 2016/007929.

2. The mannanase variant of Claim 1, wherein said variant comprises one or more variation versus SEQ ID NO:2 selected from:
P19E, T38E, D/H/N67D, F/Y129M, P168S, L/Q184L, G/H225P, K/R/Y244L, P/S/T258D, and D/E/N261R.

3. The mannanase variant of any preceding Claim, wherein said variant further comprises one or more motifs selected from a:
(i) WXₐKNDLXXAI (SEQ ID NO:11) motif at positions 31-40, wherein Xa is F or Y and X is any amino acid; (ii) LDXXXGPXGXLT (SEQ ID NO:12) motif at positions 263-274, wherein X is any amino acid; (iii) LDX₁V/AT/AGPX₂GX₃LT (SEQ ID NO:13) motif at positions 263-274, wherein X₁ is an M or L, X₂ is N, A or S and X₃ is S, T or N; and (iv) LDM/LATGPN/AG S/TLT (SEQ ID NO:14) motif at positions 263-274.

4. The mannanase variant of any preceding Claim , wherein the mannanase variant has at least 91%, 92%, 93%, 94%, 95%, 96%, 97%, 98%, or 99% amino acid sequence identity to the amino acid sequence of SEQ ID NO: 2.

5. The mannanase variant of any preceding Claim, wherein the mannanase variant has mannanase activity, wherein the mannanase activity is in the presence of a surfactant and/or a protease.

6. The mannanase variant of any preceding Claim, wherein the mannanase variant does not further comprise a carbohydrate-binding module.

7. A cleaning composition comprising the mannanase variant of any one of Claims 1-6, further comprising at least one surfactant, and optionally further comprising at least one ion selected from calcium and zinc; at least one adjunct ingredient; at least one stabilizer; from about 0.001% to about 1.0 weight % of said mannanase variant of any one of Claims 1-6; at least one bleaching agent; and/or at least one enzyme or enzyme derivative selected from acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, oxidases, pectate lyases, pectin acetyl esterases, pectinases, pentosanases, perhydrolases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, and a combination thereof.

8. The cleaning composition of Claim 7, wherein the cleaning composition:
(i) is a detergent composition selected from a laundry detergent, a fabric softening detergent, a dishwashing detergent, and a hard-surface cleaning detergent; and/or
(ii) is in a form selected from a liquid, a powder, a granulated solid, a tablet, a sheet, and a unit dose; and/or
(iii) contains phosphate or is phosphate-free and/or contains boron or is boron-free.

9. A method of cleaning comprising contacting a surface or item comprising a soil or stain comprising mannan with (i) the mannanase variant of any one of Claims 1-6, or (ii) the cleaning composition of any one of claims 7-8, wherein the mannan contained in said soil or stain is hydrolyzed.

10. The method of Claim 9, wherein said item is dishware or fabric.

11. A polynucleotide comprising a nucleic acid sequence encoding the mannanase variant of any one of Claims 1-6.

12. An expression vector comprising the polynucleotide of Claim 11.

13. A host cell comprising the expression vector of Claim 12.

14. A method for producing the mannanase variant of any one of Claims 1-6 comprising:
(a) stably transforming the host cell of Claim 13 with the expression vector of Claim 12;
(b) cultivating said transformed host cell under conditions suitable for said host cell to produce said mannanase variant; and
(c) recovering said mannanase variant.

## Patentansprüche

1. Mannanasevariante, umfassend eine Aminosäuresequenz, die eine oder mehrere Variationen gegenüber SEQ ID NO:2 umfasst, die ausgewählt sind aus:
X19E, X38E, X67D, X129M, X168S, X184L, X225P, X244L, X258D und X261R;
wobei X für eine beliebige Aminosäure steht;
wobei die Aminosäurepositionen der Variante in Entsprechung mit der Aminosäuresequenz unter SEQ ID NO:2 nummeriert sind;
wobei die Mannanasevariante eine Identität von wenigstens 90 % mit SEQ ID NO: 2 aufweist und
wobei die Mannanasevariante eine oder mehrere verbesserte Eigenschaften gegenüber einem Referenzpolypeptid mit der Aminosäuresequenz unter SEQ ID NO: 2 aufweist, wobei die verbesserte Eigenschaft aus thermischer Stabilität, Detergensstabilität, spezifischer Aktivität gegenüber einem Mannansubstrat und wie mit dem in Beispiel 1 beschriebenen Microswatch Assay bestimmter Reinigungsleistung ausgewählt ist,
mit der Maßgabe, dass die Mannanasevariante nicht die Aminosäuresequenz gemäß einer von SEQ ID NO: 62, 63, 65, 66, 67, 80 und 82 in WO 2016/007929 aufweist.

2. Mannanasevariante nach Anspruch 1, wobei die Variante eine oder mehrere Variationen gegenüber SEQ ID NO:2 umfasst, die ausgewählt sind aus:
P19E, T38E, D/H/N67D, F/Y129M, P168S, L/Q184L, G/H225P, K/R/Y244L, P/S/T258D und D/E/N261R.

3. Mannanasevariante nach einem vorhergehenden Anspruch, wobei die Variante ferner ein oder mehrere Motive umfasst, die aus einem
(i) Motiv WXₐKNDLXXAI (SEQ ID NO:11) an Position 31-40, wobei Xa für F oder Y steht und X für eine beliebige Aminosäure steht, (ii) Motiv LDXXXGPXGXLT (SEQ ID NO:12) an Position 263-274, wobei X für eine beliebige Aminosäure steht, (iii) Motiv LDX₁V/AT/AGPX₂GX₃LT (SEQ ID NO:13) an Position 263-274, wobei X₁ für ein M oder L steht, X₂ für N, A oder S steht und X₃ für S, T oder N steht, und (iv) Motiv LDM/LATGPN/AG S/TLT (SEQ ID NO:14) an Position 263-274 ausgewählt sind.

4. Mannanasevariante nach einem vorhergehenden Anspruch, wobei die Mannanasevariante eine Aminosäuresequenzidentität von wenigstens 91 %, 92 %, 93 %, 94 %, 95 %, 96 %, 97 %, 98 % oder 99 % mit der Aminosäuresequenz unter SEQ ID NO: 2 aufweist.

5. Mannanasevariante nach einem vorhergehenden Anspruch, wobei die Mannanasevariante Mannanase-Aktivität aufweist, wobei die Mannanase-Aktivität in Gegenwart eines Tensids und/oder einer Protease vorliegt.

6. Mannanasevariante nach einem vorhergehenden Anspruch, wobei die Mannanasevariante weiter kein kohlenhydratbindendes Modul umfasst,

7. Reinigungszusammensetzung, umfassend die Mannanasevariante nach einem der Ansprüche 1-6, ferner umfassend wenigstens ein Tensid und gegebenenfalls ferner umfassend wenigstens ein Ion, das aus Calcium und Zink ausgewählt ist; wenigstens einen Zusatzstoff; wenigstens einen Stabilisator; etwa 0,001 % bis etwa 1,0 Gew.-% der Mannanasevariante nach einem der Ansprüche 1-6; wenigstens ein Bleichmittel; und/oder wenigstens ein Enzym oder Enzymderivat, das aus Acyltransferasen, Amylasen, Alpha-Amylasen, Beta-Amylasen, Alpha-Galactosidasen, Arabinasen, Arabinosidasen, Arylesterasen, Beta-Galactosidasen, Beta-Glucanasen, Carrageenasen, Katalasen, Cellobiohydrolasen, Cellulasen, Chondroitinasen, Cutinasen, Endo-beta-1,4-Glucanasen, Endo-beta-Mannanasen, Exo-beta-Mannanasen, Esterasen, Exomannanasen, Galaktanasen, Glucoamylasen, Hemicellulasen, Hyaluronidasen, Keratinasen, Laccasen, Lactasen, Ligninasen, Lipasen, lipolytischen Enzymen, Lipoxygenasen, Mannanasen, Oxidasen, Pektatlyasen, Pektinacetylesterasen, Pektinasen, Pentosanasen, Perhydrolasen, Peroxidasen, Phenoloxidasen, Phosphatasen, Phospholipasen, Phytasen, Polygalacturonasen, Proteasen, Pullulanasen, Reduktasen, Rhamnogalacturonasen, Beta-Glucanasen, Tannasen, Transglutaminasen, Xylan-Acetylesterasen, Xylanasen, Xyloglucanasen, Xylosidasen, Metalloproteasen und einer Kombination davon ausgewählt ist.

8. Reinigungszusammensetzung nach Anspruch 7, wobei die Reinigungszusammensetzung:
(i) eine Waschmittelzusammensetzung ist, die aus einem Wäschewaschmittel, einem Weichspülmittel, einem Geschirrspülmittel und einem Reinigungsmittel für harte Oberflächen ausgewählt ist; und/oder
(ii) in einer Form vorliegt, die aus einer Flüssigkeit, einem Pulver, einem granulierten Feststoff, einer Tablette, einem Tuch und einer Einheitsdosis ausgewählt ist; und/oder
(iii) Phosphat enthält oder phosphatfrei ist und/oder Bor enthält oder borfrei ist.

9. Verfahren zur Reinigung, umfassend In-Kontakt-Bringen einer einen Mannan enthaltenden Schmutz oder Fleck umfassenden Oberfläche oder Sache mit (i) der Mannanase-Variante nach einem der Ansprüche 1-6 oder (ii) der Reinigungszusammensetzung nach einem der Ansprüche 7-8, wobei das in dem Schmutz oder Fleck enthaltene Mannan hydrolysiert wird.

10. Verfahren nach Anspruch 9, wobei es sich bei der Sache um Geschirr oder Textilien handelt.

11. Polynukleotid, umfassend eine Nukleinsäuresequenz, die die Mannanasevariante nach einem der Ansprüche 1-6 codiert.

12. Expressionsvektor, umfassend das Polynukleotid nach Anspruch 11.

13. Wirtszelle, umfassend den Expressionsvektor nach Anspruch 12.

14. Verfahren zur Herstellung der Mannanasevariante nach einem der Ansprüche 1-6, umfassend:
(a) stabiles Transformieren der Wirtszelle nach Anspruch 13 mit dem Expressionsvektor nach Anspruch 12;
(b) Kultivieren der transformierten Wirtszelle unter Bedingungen, die zur Produktion der Mannanasevariante durch die Wirtszelle geeignet sind; und
(c) Gewinnen der Mannanasevariante.

## Revendications

1. Variant de mannanase comprenant une séquence d'acides aminés comprenant une ou plusieurs variations par rapport à la SEQ ID NO: 2 choisies parmi :
X19E, X38E, X67D, X129M, X168S, X184L, X225P, X244L, X258D. et X261R;
X étant un quelconque acide aminé ;
les positions d'acides aminés dudit variant étant numérotées par correspondance avec la séquence d'acides aminés de la SEQ ID NO: 2 ;
le variant de mannanase ayant au moins 90 % d'identité avec la SEQ ID NO: 2, et
le variant de mannanase ayant une ou plusieurs propriétés améliorées par rapport à un polypeptide de référence ayant la séquence d'acides aminés de la SEQ ID NO: 2, la propriété améliorée étant choisie parmi la stabilité thermique, la stabilité à un détergent, une activité spécifique envers un substrat de mannane et une performance de nettoyage telle que déterminée en utilisant le dosage de microéchantillons décrit dans l'exemple 1,
à la condition que le variant de mannanase ne possède pas la séquence d'acides aminés indiquée dans l'une quelconque parmi les SEQ ID NO: 62, 63, 65, 66, 67, 80 et 82 dans le document WO 2016/007929.

2. Variant de mannanase selon la revendication 1, ledit variant comprenant une ou plusieurs variations par rapport à la SEQ ID NO: 2 choisies parmi :
P19E, T38E, D/H/N67D, F/Y129M, P168S, L/Q184L, G/H225P, K/R/Y244L, P/S/T258D, et D/E/N261R.

3. Variant de mannanase selon une quelconque revendication précédente, ledit variant comprenant en outre un ou plusieurs motifs choisis parmi un :
(i) motif WXₐKNDLXXAI (SEQ ID NO:11) au niveau des positions 31 à 40, Xa étant F ou Y et X étant un quelconque acide aminé ; (ii) motif LDXXXGPXGXLT (SEQ ID NO:12) au niveau des positions 263 à 274, X étant un quelconque acide aminé ; (iii) motif LDX₁V/AT/AGPX₂GX₃LT (SEQ ID NO:13) au niveau des positions 263 à 274, X₁ étant M ou L, X₂ étant N, A ou S et X₃ étant S, T ou N ; et (iv) motif LDM/LATGPN/AG S/TLT(SEQ ID NO:14) au niveau des positions 263 à 274,

4. Variant de mannanase selon une quelconque revendication précédente, le variant de mannanase ayant au moins 91 %, 92 %, 93 %, 94 %, 95 %, 96 %, 97 %, 98 % ou 99 % d'identité de séquence d'acides aminés avec la séquence d'acides aminés de la SEQ ID NO: 2.

5. Variant de mannanase selon une quelconque revendication précédente, le variant de mannanase ayant une activité de mannanase, l'activité de mannanase étant en la présence d'un tensioactif et/ou d'une protéase.

6. Variant de mannanase selon une quelconque revendication précédente, le variant de mannanase ne comprenant pas en outre un module de liaison à un glucide.

7. Composition de nettoyage comprenant le variant de mannanase selon l'une quelconque des revendications 1 à 6, comprenant en outre au moins un tensioactif, et éventuellement comprenant en outre au moins un ion choisi parmi calcium et zinc ; au moins un ingrédient adjuvant ; au moins un stabilisant ; d'environ 0,001 % à environ 1,0 % en poids dudit variant de mannanase selon l'une quelconque des revendications 1 à 6 ; au moins un agent de blanchiment ; et/ou au moins un(e) enzyme ou dérivé d'enzyme choisi parmi les acyltransférases, les amylases, les alpha-amylases, les bêta-amylases, les alpha-galactosidases, les arabinases, les arabinosidases, les arylestérases, les bêta-galactosidases, les bêta-glucanases, les carragénases, les catalases, les cellobiohydrolases, les cellulases, les chondroïtinases, les cutinases, les endo-bêta-1, 4-glucanases, les endobéta-mannanases, les exo-bêta-mannanases, les estérases, les exo-mannanases, les galactanases, les glucoamylases, les hémicellulases, les hyaluronidases, les kératinases, les laccases, les lactases, les ligninases, les lipases, les enzymes lipolytiques, les lipoxygénases, les mannanases, les oxydases, les pectate lyases, les pectine acétylestérases, les pectinases, les pentosanases, les perhydrolases, les peroxydases, les phénoloxydases, les phosphatases, les phospholipases, les phytases, les polygalacturonases, les protéases, les pullulanases, les réductases, les rhamnogalacturonases, les bêta-glucanases, les tannases, les transglutaminases, les xylane acétylestérases, les xylanases, les xyloglucanases, les xylosidases, les métalloprotéases, et une combinaison correspondante.

8. Composition de nettoyage selon la revendication 7, la composition de nettoyage :
(i) étant une composition de détergent choisie parmi un détergent de lessive, un détergent d'assouplissant de tissus, un détergent pour laver la vaisselle, et un détergent de nettoyage de surface dure ; et/ou
(ii) étant sous une forme choisie parmi un liquide, une poudre, un solide granulé, un comprimé, une feuille et une dose unitaire ; et/ou
(iii) contenant du phosphate ou étant exempte de phosphate et/ou contenant du bore ou étant exempte de bore.

9. Procédé de nettoyage comprenant la mise en contact d'une surface ou d'un objet comprenant des saletés ou des taches comprenant du mannane avec (i) le variant de mannanase selon l'une quelconque des revendications 1 à 6, ou (ii) la composition de nettoyage selon l'une quelconque des revendications 7 à 8, le mannane contenu dans lesdites saletés ou taches étant hydrolysé.

10. Procédé selon la revendication 9, ledit objet étant de la vaisselle ou du tissu.

11. Polynucléotide comprenant une séquence d'acides nucléiques codant pour le variant de mannanase selon l'une quelconque des revendication 1 à 6.

12. Vecteur d'expression comprenant le polynucléotide selon la revendication 11.

13. Cellule hôte comprenant le vecteur d'expression selon la revendication 12.

14. Procédé pour la production du variant de mannanase selon l'une quelconque des revendication 1 à 6 comprenant :
(a) la transformation de manière stable de la cellule hôte selon la revendication 13 avec le vecteur d'expression selon la revendication 12 ;
(b) la culture de ladite cellule hôte transformée dans des conditions appropriées pour que ladite cellule hôte produise ledit variant de mannanase ; et
(c) la récupération dudit variant de mannanase.
